# EUROPEAN PATENT APPLICATION

(11) **EP 4 483 774 A1**
(43) Date of publication of application: **01.01.2025**
(21) Application number: 23860689.1
(22) Date of filing: 25.07.2023
(51) Int. Cl.: A47L 23/20, A61L 2/07, F26B 21/00, F26B 21/02, F26B 21/08, F26B 25/12, A47B 61/04

(54) **SHOE CARE APPARATUS**

(30) Priority: 01.09.2022 KR 20220110951
(71) Applicant: LG Electronics Inc., Yeongdeungpo-gu Seoul 07336 (KR)
(72) Inventor: KIM, Ji Hun, Seoul 08592 (KR); CHOI, Kyoung Min, Seoul 08592 (KR); PARK, Hye Yong, Seoul 08592 (KR)
(74) Representative: Schornack, Oliver
(86) International application number: PCT/KR2023/010711
(87) International publication number: WO 2024/049007

(57) **Abstract**

Provided is a shoe care device in which a shoe is processed by circulating air flow. The shoe care device according to one aspect of the present invention includes: an inner cabinet having an accommodation space configured to accommodate a shoe; an outlet through which air inside the inner cabinet is suctioned; a nozzle duct hinged to the inner cabinet at one end thereof to be installed to protrude into the inner cabinet and provide an air passage; a nozzle coupled to the other end of the nozzle duct to be insertable into the shoe within the inner cabinet to spray air into the shoe; a connection path connected to the nozzle from the outlet; a blowing part installed on the connection path to blow air from the outlet toward the nozzle; and a dehumidifying part installed on the connection path to dehumidify the blown air. The nozzle duct includes: a nozzle bar provided in a tubular shape extending along a longitudinal direction and having an internal space through which air is movable; and a nozzle rib provided to connect upper and lower surfaces inside the nozzle bar to each other.

## Description

### TECHNICAL FIELD

The present invention relates to a shoes care device, and more particular, to a shoes care device which treats shoes by air circulation.

### BACKGROUND

Shoes may get wet by a wearer's sweat, external contaminants, or rain or snow. Wearing such shoes may make the wearer uncomfortable, and in the condition, germs may also breed or stink in the shoes.

Accordingly, there is an increasing interest in a shoes care device 1, which removes germs and odors by performing a predetermined treatment on the shoes so that a user can comfortably wear the shoes all the times.

For the shoes care device, Korean Patent No. 1037245 (hereinafter, referred to as "Prior Document 1") discloses "Apparatus for sterilization disposal of shoes", which includes a main body, an ultraviolet emission module, and a deodorization module.

According to Prior Document 1 above, the shoes are disposed in a sterilization chamber of the main body, and the ultraviolet emission module is driven to remove germs and odors of the shoes. Furthermore, the air in the sterilization chamber is inhaled into a ventilation pipe and discharged to the outside of the main body through an exhaust port through the deodorization module.

Here, the deodorization module includes a deodorization column made up of materials such as zeolite, activated carbon, and charcoal, and removes contaminants from the air discharged from the inside of the main body to the outside by the deodorization column.

According to Prior Document 1 above, the air from which moisture has been removed by a deodorization module including zeolite and activated carbon can be discharged to the outside of the apparatus for sterilization disposal of shoes.

However, in Prior Document 1 above, since the air is discharged to the outside of the apparatus for sterilization disposal of shoes, the air that has not sufficiently removed moisture or odors may be discharged to the outside of the apparatus for sterilization disposal of shoes, and such air may be discharged to the inside where the wearer resides.

In addition, Korean Patent Unexamined Publication No. 10-2000-0009653 (hereinafter referred to as "Prior Document 2") discloses "The shoes cabinet for sanitation", which includes a main body, a far infrared radiation part, a circulation fan, an air circulation passage, and a sanitary filter portion.

According to Prior Document 2 above, while storing shoes in shoe closet, hygiene treatments such as dehumidification, sterilization, and deodorization can be performed on the shoes by far-infrared rays and a filter during the storing of the shoes.

Here, since the sanitary filter portion is filled with excellent adsorptive materials such as charcoal, it can serve to adsorb moisture in the process of ventilating air and filter bacteria, as well as to capture malodorous substances.

According to Prior Document 2 above, the air is circulated by a circulating fan in a shoe closet, and the sanitary filter portion is disposed on a circulation path of the air to remove germs and odors in the air.

However, since Prior Document 2 above does not consider a technology to prevent the reduction of the performance of the sanitary filter portion configured to remove moisture or odors, a user may not be satisfied because the shoes are not properly treated at the time of using a shoes care device.

As described above, for the shoes care device that removes germs or odors by treating the shoes in a predetermined manner, a task that has to be solved to ensure proper performance for shoes treatment while preventing the air used for dehumidification and deodorization from being exposed to the user is present in in the process of treating the shoes.

However, there is a limitation in that the conventional shoes care device cannot properly solve such a task.

In addition, when designing and manufacturing devices including a dehumidifier such as zeolite, it is necessary to consider how the dehumidification efficiency of a dehumidifying material can be further improved, whether the dehumidifying material can be effectively regenerated, whether water vapor generated during the use of the shoes care device and the regeneration of the dehumidifying material can be effectively removed or managed, whether moisture is left in an unintended area during use of the shoes care device and the regeneration of the dehumidifying, whether components are properly disposed in a limited space, and where the devices provides excellent use convenience. There is a need to develop a shoes care device that takes these matters into consideration.

In addition, the reduction of manufacturing costs, the convenience of manufacturing and assembling each component, and the convenience of maintenance needs to be considered in the development of the shoes care device.

### DISCLOSURE

### TECHNICAL PROBLEM

An object to be achieved by the present invention is to solve the aforementioned problems caused by a shoes care device which treats shoes by air circulation.

Specifically, an object to be achieved by the present invention is to provide a shoes care device which can ensure proper performance for shoes treatment at all times by performing dehumidification and deodorization on the shoes using a dehumidifying material to refresh the shoes and regenerating the used dehumidifying material.

An object to be achieved by the present invention is to provide a shoes care device which prevents air used for dehumidification and deodorization of shoes from being exposed to a user by an air circulation structure capable of dehumidifying the air inside an inner cabinet where the shoes are placed by using a dehumidifying material and supplying the dehumidified air again to the inner cabinet.

An object to be achieved by the present invention is to provide a shoe care device in which a nozzle duct, which supplies air into the inner cabinet, is installed stably to prevent functional deterioration or inconvenience in use due to structural deformation or the like due to use.

The technical objects of the present disclosure are not restricted to those set forth Here, and other unmentioned technical objects will be clearly understood by one of ordinary skill in the art to which the present disclosure pertains by referencing the detailed description of the present disclosure given below.

### TECHNICAL SOLUTION

In order to achieve the above and other objects, a shoes care device according to one aspect of the present invention is configured not only to perform dehumidification and deodorization of shoes using a dehumidifying part but also to regenerate the used dehumidifying part. Specifically, the shoes care device is configured not only to collect moisture and bacteria in air ventilated by the dehumidifying part in a module chamber but also to heat and regenerate the dehumidifying part in the module chamber.

In addition, according to one aspect of the present invention, the shoes care device is configured such that the air used for dehumidifying and deodorizing the shoes has an air circulation structure inside the shoes care device. Specifically, a connection path through which air is circulated is formed between a outlet and a nozzle disposed inside an inner cabinet, respectively.

In addition, according to one aspect of the present invention, the shoe care device is configured such that a nozzle duct, which serves as an air passage for dehumidifying and deodorizing shoes, has sufficient rigidity to stably supply air to the inner cabinet. Specifically, a nozzle rib is provided to connect the upper and lower surfaces inside the tubular nozzle bar so as to reinforce rigidity.

In addition, according to one aspect of the present invention, the shoe care device may perform steam treatment on shoes by supplying steam into the inner cabinet.

In addition, according to one aspect of the present invention, the shoe care device may have a nozzle duct installed to face the front side from the rear wall of the inner cabinet.

In addition, according to one aspect of the present invention, in the shoe care device, the nozzle duct may be coupled to the inner cabinet in a penetration structure.

In addition, according to one aspect of the present invention, in the shoe care device, the nozzle duct may be sealed at a coupling surface other than the portion where the nozzle duct penetrates the inner cabinet, by a nozzle sealing part.

In addition, according to one aspect of the present invention, in the shoe care device, the nozzle sealing part, which is in contact with the outer peripheral surface of the nozzle bar, may be relatively thin.

In addition, according to one aspect of the present invention, in the shoe care device, the nozzle duct may be supported with respect to the inner cabinet by a nozzle supporter.

In addition, according to one aspect of the present invention, in the shoe care device, a nozzle supporter hook provided on the nozzle supporter may be fastened to the outer peripheral surface of the nozzle bar.

In addition, according to one aspect of the present invention, the shoe care device may have a nozzle supporter rib provided on the inner surface of the nozzle supporter along the longitudinal direction of the nozzle bar.

In addition, in the shoe care device according to one aspect of the present invention, the nozzle supporter may be supported on the inner cabinet by a hinge axis resistance part by being hinged to the inner cabinet via a nozzle supporter hinge axis.

In addition, in the shoe care device according to one aspect of the present invention, the hinge axis resistance part may generate a frictional force on the nozzle supporter hinge axis.

In addition, in the shoe care device according to one aspect of the present invention, a pair of nozzle supporter hinge axes may be installed on opposite side surfaces of the nozzle supporter.

In addition, in the shoe care device according to one aspect of the present invention, the hinge axis resistance part may include a first hinge axis holder and a second hinge axis holder.

In addition, in the shoe care device according to one aspect of the present invention, a hinge axis holder hook provided on the second hinge axis holder may be fastened to a hinge axis holder groove provided in the first hinge axis holder.

In addition, in the shoe care device according to one aspect of the present invention, one end and the other end of the nozzle duct may be hinged to the inner cabinet and the nozzle, respectively, and separately from the nozzle duct, one end and the other end of the nozzle connector are hinged to the inner cabinet and the nozzle, respectively.

In addition, in the shoe care device according to one aspect of the present invention, the upper surface of the nozzle connector may be engaged with a nozzle supporter groove provided in the lower surface of the nozzle supporter.

The technical objects of the present disclosure are not restricted to those set forth Here, and other unmentioned technical objects will be clearly understood by one of ordinary skill in the art to which the present disclosure pertains by referencing the detailed description of the present disclosure given below.

### BRIEF DESCRIPTION OF THE DRAWINGS

FIG. 1a is a perspective view illustrating a shoes care device according to one embodiment of the present invention.
FIG. 1b is a perspective view of the shoes care device of FIG. 1a, when viewed from another direction, illustrating a state in which a door is opened.
FIG. 2a is a perspective view illustrating a state in which a part of the door and an outer cabinet are removed from the shoes care device of FIG. 1b.
FIG. 2b is a perspective view illustrating a state in which the shoes care device illustrated in FIG. 2a is viewed from another direction.
FIG. 3 is a front view illustrating a state in which a door is removed from the shoes care device illustrated in FIG. 1b. FIG. 3 illustrates shoes accommodated in an inner cabinet together.
FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.
FIG. 5 is a perspective view illustrating a state in which the door, the outer cabinet, and the inner cabinet are removed from the shoes care device according to one embodiment of the present invention.
FIG. 6 is a perspective view illustrating a part of a machine room of the shoes care device illustrated in FIG. 5.
FIG. 7a is a view illustrating a steam valve according to one embodiment of the present invention, and illustrating a connection relationship considering the movement of steam.
FIG. 7b is an exploded perspective view illustrating the steam valve illustrated in FIG. 7a.
FIG. 8a is a cross-sectional view taken along line D-D' of the shoes care device illustrated in FIG. 3. FIG. 8a illustrates a state in which the door is included in the shoes care device, and does not illustrate the shoes.
FIG. 8b is a view illustrating a state in which a main shelf is removed from the shoes care device illustrated in FIG. 8a.
FIG. 9 is a cross-sectional view taken along line E-E' of the shoes care device illustrated in FIG. 3. FIG. 9 illustrates a state in which the door is included in the shoes care device.
FIG. 10a is an exploded perspective view illustrating a drying module in the shoes care device according to one embodiment of the present invention. FIG. 10b is an exploded perspective view illustrating a partial configuration of the drying module at a part to which a damper is coupled.
FIG. 11 is a diagram illustrating a connection relationship between components and a flow of fluid in the shoes care device according to one embodiment of the present invention.
FIG. 12 is a view illustrating in more detail a nozzle duct and a nozzle installed in the inner cabinet in the shoe care device according to one embodiment of the present invention.
FIG. 13 is a cross-sectional view illustrating coupling structures at one end and the other end of the nozzle duct of FIG. 12.
FIG. 14 is a cross-sectional view illustrating a portion where the nozzle duct of FIG. 12 is coupled to the inner cabinet.
FIG. 15 is a cross-sectional view illustrating a portion where the nozzle duct of FIG. 12 is coupled to the nozzle.
FIGS. 16 and 17 are views illustrating the state in which the angle of the nozzle duct of FIG. 12 is adjusted.
FIG. 18 is a cross-sectional view illustrating the interior of the nozzle duct of FIG. 12.
FIG. 19 is a view illustrating the nozzle duct and the nozzle of FIG. 12 viewed in another direction.
FIG. 20 is a view illustrating the nozzle duct and the nozzle of FIG. 12 viewed in another direction.
FIG. 21 is an exploded view illustrating the nozzle duct and the nozzle of FIG. 20.
FIG. 22 is a cross-sectional view illustrating the interior of the nozzle duct of FIG. 20.
FIGS. 23 and 24 are views illustrating the nozzle sealing part of FIG. 20 in more detail.
FIGS. 25 to 28 are views illustrating the nozzle supporter of FIG. 20 in more detail.
FIGS. 29 to 31 are views illustrating the hinge axis resistance part of FIG. 20 in more detail.

### DETAILED DESCRIPTION

Hereinafter, exemplary embodiments disclosed herein will be described in detail with reference to the accompanying drawings, and like reference numerals designate like elements, and redundant description thereof will be omitted. Suffixes "module" and "unit or portion" for elements used in the following description are merely provided for facilitation of preparing this specification, and thus they are not granted a specific meaning or function. Wherever possible, the same reference numbers will be used throughout the drawings to refer to the same or like parts. In the following description, known functions or structures, which may confuse the substance of the present disclosure, are not explained. The accompanying drawings are used to help easily explain various technical features and it should be understood that the exemplary embodiments presented herein are not limited by the accompanying drawings. As such, the present disclosure should be construed to extend to any alterations, equivalents and substitutes in addition to those which are particularly set out in the accompanying drawings.

Although the terms first, second, and the like, may be used herein to describe various elements, these elements should not be limited by these terms. These terms are generally only used to distinguish one element from another.

When an element or layer is referred to as being "on," "engaged to," "connected to," or "coupled to" another element or layer, it may be directly on, engaged, connected, or coupled to the other element or layer, or intervening elements or layers may be present. In contrast, when an element is referred to as being "directly on," "directly engaged to," "directly connected to," or "directly coupled to" another element or layer, there may be no intervening elements or layers present.

The singular expressions include plural expressions unless the context clearly dictates otherwise.

It should be understood that the terms "comprises," "comprising," "includes," "including," "containing," "has," "having" or any other variation thereof specify the presence of stated features, integers, steps, operations, elements, and/or components, but do not preclude the presence or addition of one or more other features, integers, steps, operations, elements, and/or components.

A first direction (X direction), a second direction (Y direction), and a third direction (Z direction) described in one embodiment of the present invention may be directions orthogonal to each other.

Each of the first direction (X direction) and the second direction (Y direction) may be a direction parallel to the horizontal direction, and the third direction (Z direction) may be a direction parallel to the vertical direction. When the first direction (X direction) is parallel to a left-right direction, the second direction (Y direction) may be parallel to a front-rear direction. When the first direction (X direction) is parallel to the front-rear direction, the second direction (Y direction) may be parallel to the left-right direction.

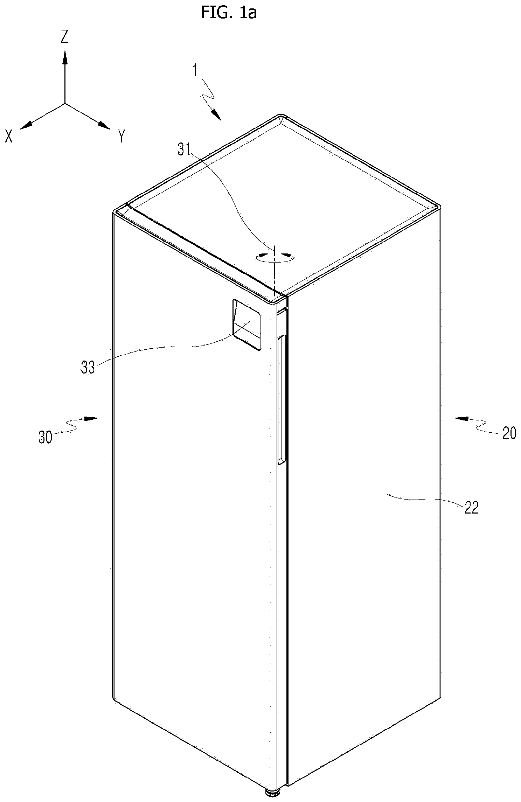

FIG. 1a is a perspective view illustrating a shoes care device according to one embodiment of the present invention.

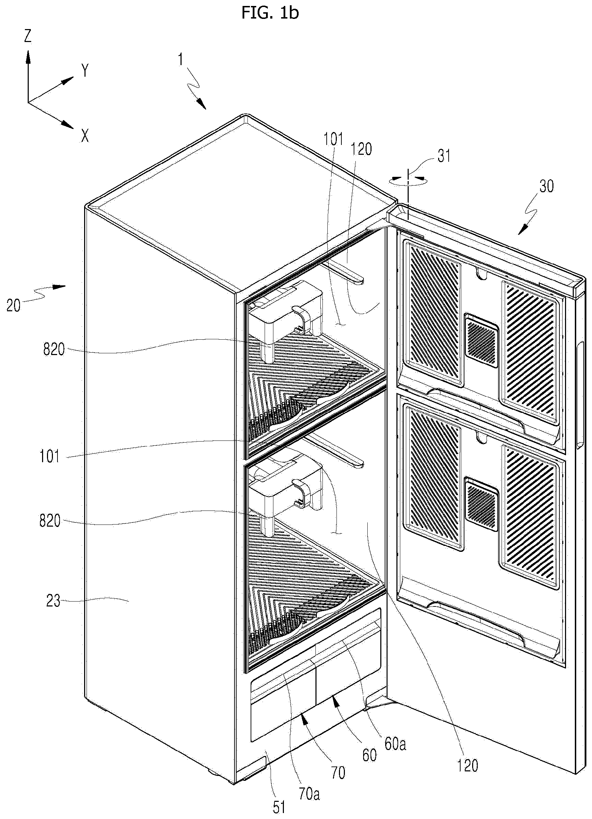

FIG. 1b is a perspective view of the shoes care device of FIG. 1a, when viewed from another direction, illustrating a state in which a door is opened.

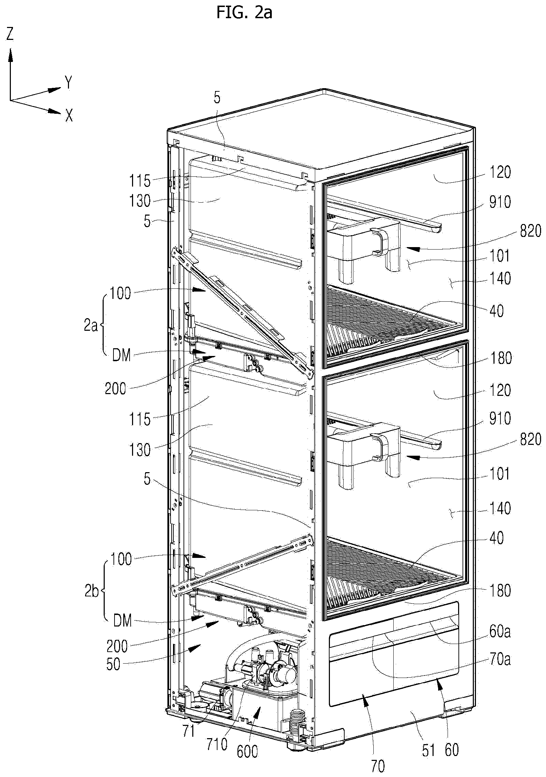

FIG. 2a is a perspective view illustrating a state in which a part of the door and an outer cabinet are removed from the shoes care device of FIG. 1b.

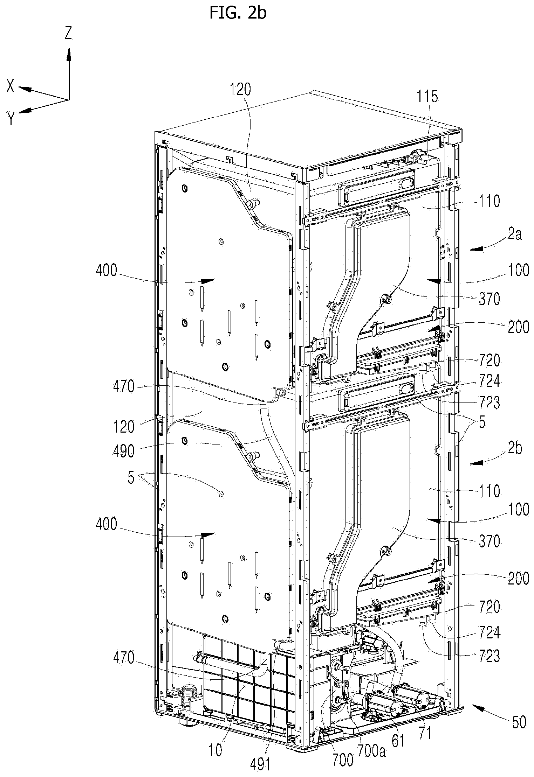

FIG. 2b is a perspective view illustrating a state in which the shoes care device illustrated in FIG. 2a is viewed from another direction.

A shoes care device 1 according to one embodiment of the present invention may include an outer cabinet 20, a door 30, an inner cabinet 100, and a machine room 50. The shoes care device 1 may include a main frame 5.

The outer cabinet 20 and the door 30 may form an overall appearance of the shoes care device 1. The outside of the shoes care device 1 may be formed in a hexahedral shape. That is, while the outer cabinet 20 and the door 30 are coupled to each other and the door 30 is closed, the appearance of the shoes care device 1 may be formed in a hexahedral shape. However, the shoes care device 1 according to one embodiment of the present invention is not limited to such a shape and may have various three-dimensional shapes.

When the door 30 forms the front of the shoes care device 1, the outer cabinet 20 may form an upper side surface, a left-side surface, a right-side surface, a rear surface, and bottom surfaces of the shoes care device 1.

The main frame 5 may form an overall framework of the shoes care device 1. The main frame 5 may have a hexahedral structure.

The outer cabinet 20 may be detachably fixed to the main frame 5.

The outer cabinet 20 may include an outer rear plate 21, a first outer side plate 22, and a second outer side plate 23.

The outer back plate 21, the first outer side plate 22, and the second outer side plate 23 may be formed integrally with each other, or the outer back plate 21, the first outer side plate 22, and the second outer side plate 23 may be individually formed.

The outer rear plate 21 forms a vertically erected wall surface. The outer rear plate 21 may form a surface orthogonal to the first direction (X direction). The outer rear plate 21 may form a rear wall surface in the first direction (X direction) on the outer cabinet 20. The outer rear plate 21 may form a rear surface in the first direction (X direction) in the shoes care device 1. The outer rear plate 21 may form an entire outer rear surface of the shoes care device 1.

The first outer side plate 22 and the second outer side plate 23 form vertically erected wall surfaces, respectively, and form opposing wall surfaces facing each other.

The first outer side plate 22 is disposed at any one side with respect to a reference plane RP that is parallel to the first direction (X direction) as a horizontal direction and is a vertical plane. The second outer side plate 23 is disposed on the opposite side of the first outer side plate 22 with respect to the reference plane RP. The first outer side plate 22 may form a left wall surface of the outer cabinet 20, and the second outer side plate 23 may form a right wall surface of the outer cabinet 20.

The outer cabinet 20 may be disposed outside the inner cabinet 100 and the machine room 50 to form an outer wall surface of the machine room 50. When a separate cabinet for the machine room 50 is not provided in the shoes care device 1, the outer cabinet 20 may form a wall separating the machine room 50 from the outside.

The door 30 is configured to open and close the inside of the shoes care device 1. The door 30 may form any one surface of the shoes care device 1. The door 30 may form a left side or a right side of the shoes care device 1, or may form a front side of the shoes care device 1.

In the shoes care device 1, the door 30 may be hinge-coupled.

In one embodiment, the door 30 may be hinge-coupled to the main frame 5. In another embodiment, the door 30 may be hinge-coupled to the outer cabinet 20, and in another embodiment, the door 30 may be hinge-coupled to the inner cabinet 100 and/or the machine room 50.

A hinge rotation axis 31 of the door 30 may be formed in the vertical direction. That is, in the shoes care device 1, the door 30 may be configured to be rotatable bidirectionally around a rotation axis 31 in the vertical direction.

In one embodiment, the shoes care device 1 may include one door 30. In another embodiment, the shoes care device 1 may include two or more doors.

When two doors are provided in the shoes care device 1, each door may be individually rotated around each rotation axis.

The first direction (X direction) described in one embodiment of the present invention may be parallel to or substantially parallel to the horizontal direction.

In one embodiment, the first direction (X direction) may be a direction from the rear of the shoes care device 1 to the front.

Hereinafter, except for a particularly limited case, it will be described that the door 30 is formed in a front side of the shoes care device 1. That is, a surface on which the door 30 is formed in the shoes care device 1 is described as a front surface of the shoes care device 1.

Furthermore, hereinafter, except for a particularly limited case, it will be described that the first direction (X direction) is parallel to the front-rear direction, the second direction (Y direction) is parallel to the left-right direction, and the third direction (Z direction) is parallel to the vertical direction.

The inner cabinet 100 and the machine room 50 may be provided inside the outer cabinet 20.

The inner cabinet 100 may be formed in a box shape, and a predetermined space may be formed therein. The space inside the inner cabinet 100 forms an accommodation space 101, and shoes S may be accommodated in the accommodation space 101.

A plurality of shoes S may be disposed together in the accommodation space 101 of one inner cabinet 100.

The inner cabinet 100 may be fixed to the main frame 5.

The inner cabinet 100 has a predetermined size along the first direction (X direction), the second direction (Y direction), and the third direction (Z direction).

The inner cabinet 100 is formed in the shape of a box opened to any one side. The inner cabinet 100 may be formed in a form opened to the front side of the shoes care device 1. The inner cabinet 100 may be configured to include a main opening 140. The main opening 140 may be provided to open the front side of the inner cabinet 100 in the first direction (X direction). The shoes may be disposed inside the inner cabinet 100 or withdrawn from the inner cabinet 100 through the main opening 140.

The main opening 140 of the inner cabinet 100 may be closed or opened by the door 30.

The inner cabinet 100 may include an inner rear plate 110, a first inner side plate 120, a second inner side plate 130, and an inner upper plate 115.

The inner rear plate 110, the first inner side plate 120, the second inner side plate 130, and the inner upper plate 115 may be formed integrally with each other. The inner cabinet 100 may be made of a single material and be formed by injection molding.

The inner rear plate 110 forms a vertically erected wall surface. The inner rear plate 110 may form a surface orthogonal to the first direction (X direction). The inner rear plate 110 may form a rear wall surface of the inner cabinet 100 in the first direction (X direction). The inner rear plate 110 may be formed parallel to the outer rear plate 21.

The first inner side plate 120 and the second inner side plate 130 form vertically erected wall surfaces, respectively, and form opposing wall surfaces facing each other.

The first inner side plate 120 is dispose on either side with respect to the reference plane RP that is parallel to the first direction (X direction) as the horizontal direction and is a vertical surface. The second inner side plate 130 is disposed on the opposite side of the first inner side plate 120 with respect to the reference plane RP. The first inner side plate 120 forms a left wall surface of the inner cabinet 100, and the second inner side plate 130 forms a right wall surface of the inner cabinet 100.

The first inner side plate 120 may be formed parallel to the first outer side plate 22, and the second inner side plate 130 may be formed parallel to the second outer side plate 23.

In one embodiment of the present invention, the inner cabinet 100 may have the form where a lower part thereof is opened. Accordingly, the inner cabinet 100 includes a lower opening 150 formed by opening the lower part thereof. When the inner cabinet 100 is formed in a hexahedral shape on the whole, the lower opening 150 may be formed large to form all or most of a lower surface of the inner cabinet 100 (see FIGS. 53 and 54).

However, the shoes care device 1 according to one embodiment of the present invention is not used in a state in which an entire lower part of the inner cabinet 100 is opened, but is used in a state in which the inner cabinet 100 and a module housing 200 are coupled to each other so that the lower opening 150 of the inner cabinet 100 is shielded by the module housing 200. That is, the shoes care device 1 is used so that an upper surface of the module housing 200 forms a bottom surface of the accommodation space 101 of the inner cabinet 100. A further explanation thereof will be described below.

A main shelf 40 may be provided in the inside 101 of the inner cabinet 100. The main shelf 40 may be formed such that the shoes S are settled on an upper surface thereof.

The main shelf 40 may be formed in the form of a plate with a predetermined area, or may be formed in the form of a grill where multiple bars are spaced apart from each other.

One main shelf 40 may be provided, or a plurality of main shelves 40 may be provided.

The main shelf 40 may have a substantially flat plate shape and be disposed on a bottom surface of the inner cabinet 100. The main shelf 40 is settled on an upper side of a module cover 202 of the inner cabinet 100. The main shelf 40 may be disposed on the upper side of the module cover 202 of the inner cabinet 100 in a stacked form.

The main shelf 40 is detachable from the inner cabinet 100, and when the main shelf 40 is withdrawn from the inner cabinet 100, the upper surface of the module housing 200 is exposed.

The main shelf 40 may have a rectangular shape when viewed in a plan view. The size of the main shelf 40 may be a size corresponding to the bottom of the accommodation space 101 of the inner cabinet 100. That is, when the main shelf 40 is disposed inside the inner cabinet 100, the main shelf 40 may form all or most of the bottom of the accommodation space 101 of the inner cabinet 100.

In one embodiment, the machine room 50 may be provided in a lower side of the inner cabinet 100. In the machine room 50, some of the components that constitutes the shoes care device 1 may be accommodated, and, in this case, the components that are accommodated in the machine room 50 may be fixed to a main frame 5 or to the inner cabinet 100 or the outer cabinet 20.

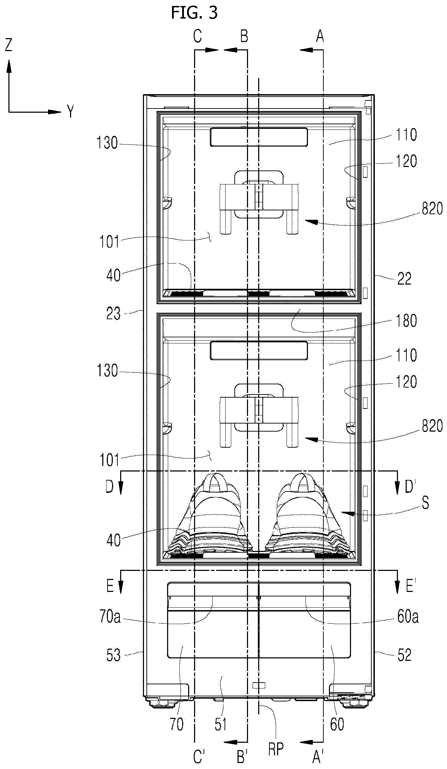

FIG. 3 is a front view illustrating a state in which a door is removed from the shoes care device illustrated in FIG. 1b. FIG. 3 illustrates shoes accommodated in an inner cabinet together.

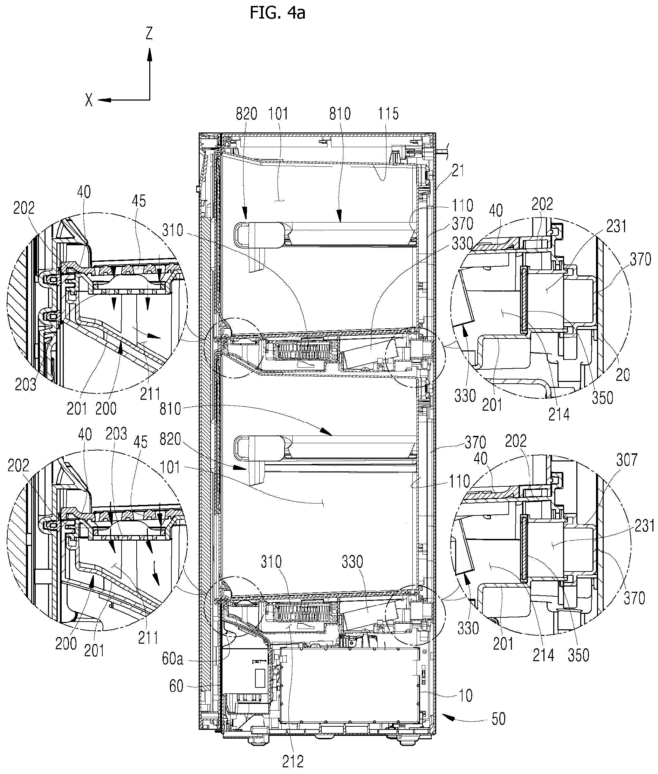

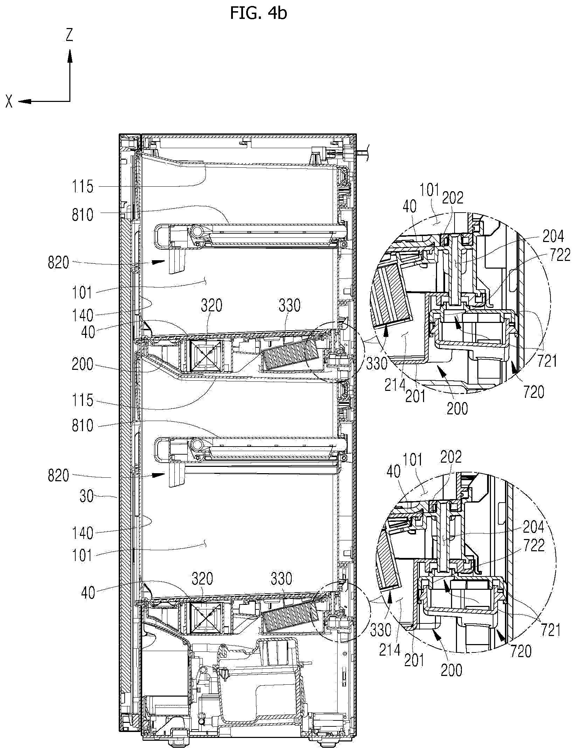

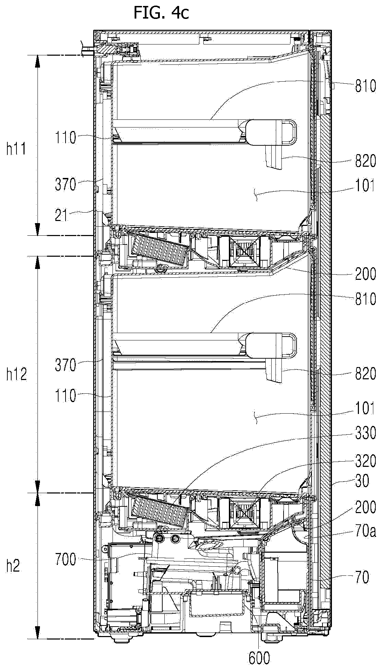

FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.

The shoes care device 1 according to one embodiment of the present invention includes management devices 2a and 2b. In the shoes care device 1, a plurality of management devices 2a and 2b may be provided. In one embodiment, the shoes care device 1 may include a first management device 2a and a second management device 2b. That is, the shoes care device 1 may include two separate management devices 2a and 2b.

The 'management device' described in the present invention may refer to a 'first management device 2a' and a 'second management device 2b', respectively, except for a particularly limited case.

The management devices 2a and 2b include the above-described inner cabinet 100.

In one embodiment of the present invention, since the inner cabinet 100 of the first management device 2a and the inner cabinet 100 of the second management device 2b may be distinguished from each other, the inner cabinet 100a of the first management device 2a may refer to a first inner cabinet 100a, and inner cabinet 100a of the second management device 100b may refer to a second inner cabinet 100b.

It may be understood that the 'inner cabinet 100' described in one embodiment of the present invention refers to each of the 'first inner cabinet 100a' and the 'second inner cabinet 100b', except for a particularly limited case.

When the door 30 is closed in the shoes care device 1, the door 30 closes the main opening 140 of the first management device 2a and also closes the main opening 140 of the second management device 2b. That is, the door 30 may simultaneously seal the accommodation space 101 of the inner cabinet 100 of the first management device 2a and the accommodation space 101 of the inner cabinet 100 of the second management device 2b. In this case, the accommodation space 101 of the inner cabinet 100 of the first management device 2a and the accommodation space 101 of the inner cabinet 100 of the second management device 2b may be configured such that they do not communicate with each other.

As described above, in one embodiment of the present invention, the accommodation space 101 of the inner cabinet 100 of the first management device 2a and the accommodation space 101 of the inner cabinet 100 of the second management device 2b may form independent spaces, and may form blocked spaces (not communicating with each other). Accordingly, the temperature and humidity of the accommodation space 101 of the first management device 2a and the temperature and humidity of the accommodation space 101 of the second management device 2b may be controlled differently from each other.

The management devices 2a and 2b may be configured to include a connection path F10. The management devices 2a and 2b may be configured to include a blowing part 310 and a dehumidifying part 330. The management devices 2a and 2b may include a outlet 203 and a nozzle 820.

The management devices 2a and 2b may include the module housing 200 forming a connection path F10.

The management devices 2a and 2b may include a regeneration path F20. The management devices 2a and 2b may include a heating part 320.

The management devices 2a and 2b may include a conversion flow path F10a.

The management devices 2a and 2b may include a damper 350.

The shoes care device 1 may include a steam generator 700 and a steam valve 710.

The shoes care device 1 may include a sump 600, a water supply tank 60, and a drain tank 70.

Since all or part of the outer cabinet 20 may be spaced apart from the inner cabinet 100, a predetermined gap may be formed between the inner cabinet 100 and the outer cabinet 20.

In a space between the inner cabinet 100 and the outer cabinet 20, components constituting the shoes care device 1 may be provided, and various flow paths constituting the shoes care device 1 may be provided. In one embodiment of the present invention, part of the connection path F10 may be provided between the inner cabinet 100 and the outer cabinet 20, and part of the regeneration path F20 may be provided between the inner cabinet 100 and the outer cabinet 20.

A dry air duct 370 forming the connection path F10 may be provided between the outer rear plate 21 and the inner rear plate 110. Furthermore, a condenser 400 forming the regeneration path F20 may be provided between the outer rear plate 21 and the inner rear plate 110.

The connection path F10 forms a flow path of a fluid.

The connection path F10 forms a passage through which air and/or condensed water inside the shoes care device 1 moves.

The dehumidifying part 330 is disposed inside the connection path F10 and includes a dehumidifying material. The dehumidifying part 330 may be entirely formed of the dehumidifying material, or a part thereof may be formed of the dehumidifying material. A further explanation of the dehumidifying part 330 will be described below.

According to one embodiment of the present invention, the shoes care device 1 has an air circulation structure in which the air inside the inner cabinet 100 in which the shoes are disposed is sucked into the connection path F10 to dehumidify the air using a dehumidifying material 331 and the dehumidified air may be supplied back into the inner cabinet 100.

The connection path F10 may be used as a means to achieve such an air circulation structure in the shoes care device 1. All or part of the connection path F10 may be formed of a pipe, a hose, a tube, a duct, a housing, or a combination thereof.

The module housing 200 according to one embodiment of the present invention forms part of the connection path F10.

The outlet 203 is formed in the module housing 200. The outlet 203 communicates with the accommodation space 101 of the inner cabinet 100, and forms an inlet of the module housing 200 through which the air of the accommodation space 101 of the inner cabinet 100 is suctioned into the module housing 200. The outlet 203 may be disposed below the main shelf 40. In this case, the main shelf 40 may be formed so as not to block the air in the accommodation space 101 from being sucked into the outlet 203. To this end, when the main shelf 40 is formed in a plate shape, a plurality of holes 45 penetrating in the vertical direction may be formed in the main shelf 40 so as to move the air.

In the shoes care device 1, the inner cabinet 100 and the machine room 50 may form a space separated from each other. Furthermore, the module housing 200 that is part of the management devices 2a and 2b may be provided between the inner cabinet 100 and the machine room 50.

The inner cabinet 100, the module housing 200, and the machine room 50 are provided inside the shoes care device 1 according to one embodiment of the present invention.

The inner cabinet 100, the module housing 200, and the machine room 50 may be continuously arranged from the upper side to the lower side. When the shoes care device 1 according to one embodiment of the present disclosure includes the first management device 2a and the second management device 2b, the first management device 2a may be disposed above the second management device 2b. That is, the first management device 2a, the second management device 2b, and the machine room 50 may be continuously arranged from the upper side to the lower side.

Since the first management device 2a and the second management device 2b include the inner cabinet 100 and the module housing 200, respectively, they may be continuously arranged in an order of the inner cabinet 100 of the first management device 2a, the module housing 200 of the first management device 2a, the inner cabinet 100 of the second management device 2b, the module housing 200 of the second management device 2b, the machine room 50 from the upper side to the lower side.

The inner cabinet 100 may form a space that mainly accommodates an article (shoes S) to be managed, and the module housing 200 and the machine room 50 may form a space that mainly accommodates components for an operation of the shoes care device 1.

In the shoes care device 1 according to one embodiment of the present invention, the blowing part 310, the dehumidifying part 330 (and the dehumidifying material 331), and the heating part 320 may be accommodated inside the module housing 200.

In addition, the machine room 50 may be configured to accommodate a controller 10, the sump 600, the steam generator 700, and the steam valve 710 therein. Furthermore, the machine room 50 may be configured to accommodate the water supply tank 60 and the drain tank 70.

Among the components constituting the management devices 2a and 2b, components not included in the module housing 200 may be fixedly coupled to the inner cabinet 100 and the outside of the module housing 200 or may be fixedly coupled to the main frame 5.

Components coupled to or accommodated in the machine room 50 may be fixedly coupled to the machine room 50.

The machine room 50 may include a first wall 51.

A first wall 51 forms one wall surface of the machine room 50. The first wall 51 may be erected in the vertical direction, or may be erected in the substantially vertical direction. In one embodiment, the first wall 51 may form a wall surface orthogonal to or inclined with the first direction (X direction).

The first wall 51 may form a front wall surface of the machine room 50, a left wall of the machine room 50, or a right wall of the machine room 50.

The machine room 50 may include a second wall 52 and a third wall 53. The second wall 52 and the third wall 53 form opposite wall surfaces facing each other in the machine room 50. The second wall 52 and the third wall 53 may be erected in the vertical direction, or may be erected in the substantially vertical direction.

When the first wall 51 forms a front wall of the machine room 50, the second wall 52 may form a left wall of the machine room 50, and the third wall 53 may form a right wall of the machine room 50.

The first wall 51 may be formed integrally with the inner cabinet 100, and the second wall 52 and the third wall 53 may be formed integrally with the outer cabinet 20, respectively.

Each of the water supply tank 60 and the drain tank 70 may be formed in the form of a container for accommodating water.

The water supply tank 60 may be configured to store water supplied into the shoes care device 1 inside. The water supply tank 60 may be configured to store water supplied into the steam generator 700 inside.

In order to supply water from the water supply tank 60 into the shoes care device 1, a water pump 61 may be connected to the water supply tank 60. The water supply tank 60 for moving water and the first water pump 61 may be connected by a pipe, a hose, etc.

The drain tank 70 may be configured to store water discharged from the shoes care device 1 inside. The drain tank 70 may store water condensed inside the shoes care device 1. The drain tank 70 may be configured to store water drained from the sump 600.

In order to discharge water to the drain tank 70, a water pump 71 (a second water pump) may be connected to the drain tank 70. The drain pipe 70 for moving water and the second water pump 71 may be connected by a pipe, a hose, etc.

The water supply tank 60 and the drain tank 70 may be coupled to the machine room 50 to get exposed from the outside of one wall surface of the machine room 50.

The water supply tank 60 and the drain tank 70 may be disposed in front of the machine room 50.

The water supply tank 60 and the drain tank 70 may form one wall surface of the machine room 50 along with the first wall 51. When the first wall 51 forms a front surface of the machine room 50, the water supply tank 60 and the drain tank 70 may be exposed from a front side of the machine room 50, and may be coupled to the machine room 50 to get exposed from the outside of the first wall 51.

As the water supply tank 60 and the drain tank 70 are exposed to the outside of the first wall 51, a user may inject water into the water supply tank 60 or discharge water from the drain tank 70.

The water supply tank 60 and the drain tank 70 may be configured to be detachable from the machine room 50. The water supply tank 60 and the drain tank 70 may be detached from the first wall 51. In order to facilitate the attachment and detachment of the water supply tank 60 and the drain tank 70, a handle 60a of the water supply tank 60 may be formed on an outer surface of the water supply tank 60, and a handle 70a of the drain tank 70 may be formed on an outer surface of the drain tank 70.

Each of the water supply tank 60 and the drain tank 70 may be configured to be separated from the machine room 50 in an outer direction of the first wall 51.

The controller 10 may be configured to control operations of each component in connection with each component constituting the shoes care device 1.

In order to control the controller 10, the shoes care device 1 may be provided with a storage medium in which an application program is stored, and the controller 10 may be configured to control the shoes care device 1 by driving an application program according to information input to the shoes care device 1 and information output from the shoes care device 1.

The controller 10 may control the first management device 2a and the second management device 2b constituting the shoes care device 1 to operate individually. The controller 10 may control the first management device 2a and the second management device 2b to operate in different states, and may control shoes (e.g., sneakers) in the first management device 2a and shoes (e.g., heels) in the second management device 2b to be managed under different conditions. Furthermore, the controller 10 may control the first management device 2a and the second management device 2b to interwork with each other.

The door 30 may be disposed on either the inner cabinet 100 or the machine room 50 on the same side as the first wall 51. When the door 30 forms the front surface of the shoes care device 1, the first wall 51 forms the front surface of the machine room 50, and the door 30 is disposed directly outside the first wall 51.

In one embodiment of the present invention, the door 30 may be configured to open and close the inner cabinet 100 and further expose or shield the front surface of the machine room 50.

The door 30 may be configured to expose or shield the inner cabinet 100, the water supply tank 60, and the drain tank 70.

As described above, in the shoes care device 1, the door 30, the water supply tank 60, and the drain tank 70 are formed on the same side, and when the door 30 is opened, the water supply tank 60 and the drain tank 70 may be exposed and separated from the shoes care device 1.

In the arrangement explained above, even if left and right sides and a rear side of the shoes care device 1 are blocked by other goods or structures, the door 30 may be opened on a front side of the shoes care device 1, and the water supply tank 60 and the drain tank 70 can be separated from or coupled again to the shoes care device 1.

A control panel 33 for controlling the shoes care device 1 is provided on an outer side of the door 30. The control panel 33 may be formed of a touch screen. A control unit (controller 10) is provided in the inner space of the door 30 to control each component of the shoes care device 1 in connection with the control panel 33. The controller 10 may be provided inside the machine room 50.

As illustrated in FIGS. 1a and 1b, in one embodiment, the door 30 may be configured to simultaneously expose or shield the inner cabinet 100 and the machine room 50.

In another embodiment, the door 30 may be configured to open and close only the inner cabinet 100. In this case, the machine room 50 may not be shielded by the door 30. Furthermore, in this case, the shoes care device 1 according to one embodiment of the present invention may be further provided with a dedicated door of the machine room 50 to open and close the machine room 50 separately from the door 30.

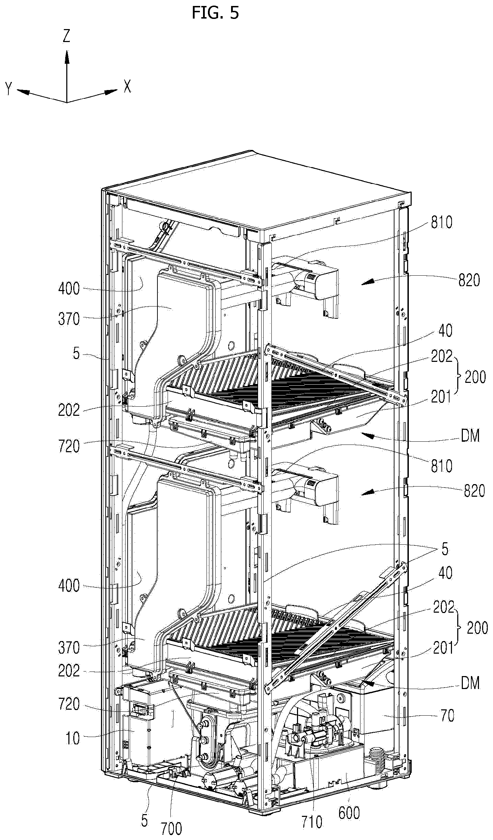

FIG. 5 is a perspective view illustrating a state in which the door 30, the outer cabinet 20, and the inner cabinet 100 are removed from the shoes care device 1 according to one embodiment of the present invention.

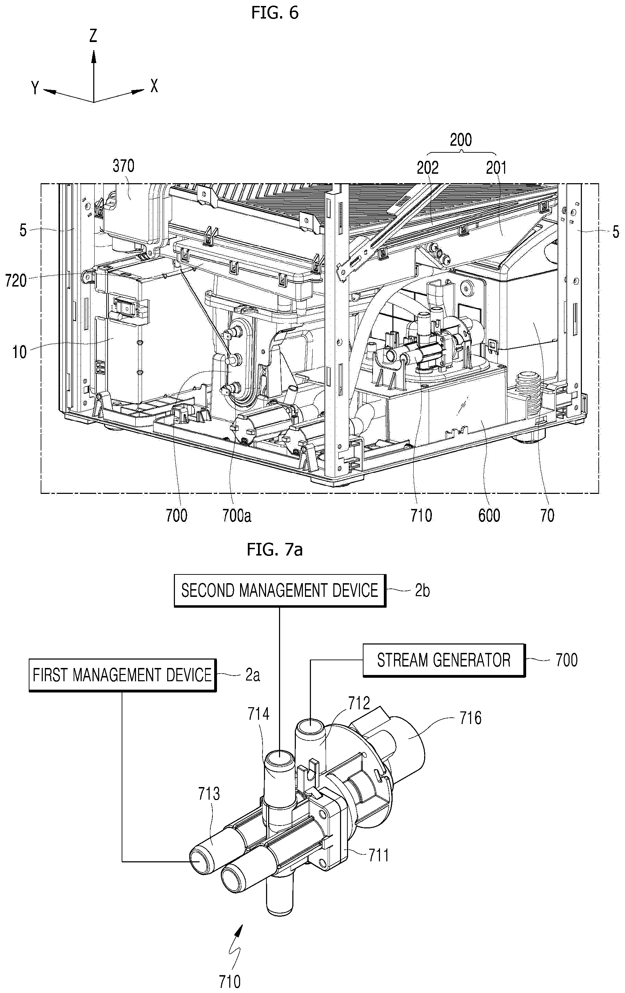

FIG. 6 is a perspective view illustrating a machine room part of the shoes care device 1 illustrated in FIG. 5.

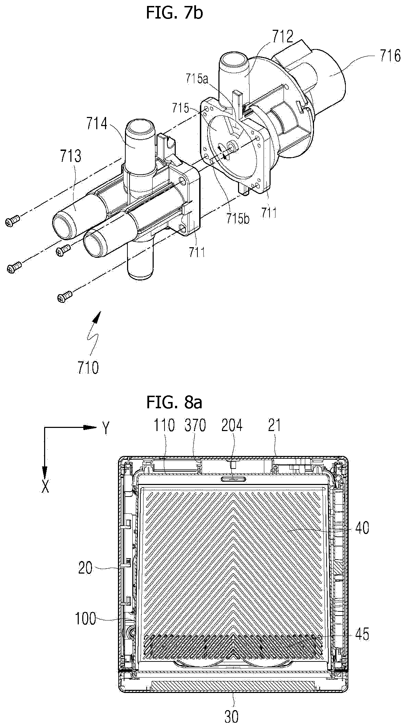

FIG. 7a is a view illustrating the steam valve 710 according to one embodiment of the present invention, and illustrating a connection relationship considering the movement of steam. FIG. 7b is an exploded perspective view illustrating the steam valve illustrated in FIG. 7a.

The shoes care device 1 is provided with the steam generator 700 as a device configured so as to generate moisture inside the inner cabinet 100. The steam generator 700 may be provided inside the machine room 50. The steam generator 700 is configured to generate steam and selectively supply moisture and steam to the inside of the inner cabinet 100.

The shoes care device 1 according to one embodiment may be provided with one steam generator 700, and the shoes care device 1 according to another embodiment may be provided with two or more steam generators 700.

When the shoes care device 1 is provided with one steam generator 700, the steam generator 700 may be configured to supply steam into the inner cabinet 100 of the first management device 2a and/or into the inner cabinet 100 of the second management device 2b.

When the shoes care device 1, one steam generator 700 may be provided with two or more steam generators 700, one of the steam generators 700 may supply steam to the inner cabinet 100 of the first management device 2a, and the other steam generator 700 may supply steam to the inner cabinet 100 of the second management device 2b.

Moist air formed by the steam generator 700 ('air' described in one embodiment of the present invention may be 'air including moisture') is supplied toward the accommodation space 101 of the inner cabinet 100, and moisture may be circulated in the accommodation space 101 of the inner cabinet 100, and accordingly, the moisture may be supplied to the shoes S.

The shoes care device 1 according to one embodiment of the present invention may be a refresher device that refreshes the shoes.

Here, refreshing may refer to a process of removing contaminants, deodorizing, sanitizing, preventing static electricity, or warming by supplying air, heated air, water, mist, steam, etc. to the shoes.

The steam generator 700 may supply steam to the accommodation space 101 of the inner cabinet 100 in which the shoes S are accommodated, and may perform steam treatment on the shoes, which is further meant to exert a refreshing effect due to swelling of shoes materials as well as sterilization by high-temperature steam.

The steam generator 700 is provided with a separate heater 700a that heats an inner space and water in the inner space and is configured to heat water to generate steam and supply the heated water to the accommodation space 101 of the inner cabinet 100.

An external faucet, etc., as a water supply source for supplying water to the steam generator 700, may be used, or a container-type water supply tank provided on one side of the machine room 50 may be used. The steam generator 700 may generate steam by receiving water from the water supply tank 60.

The water supply tank 60 for the movement of water and the steam generator 700 may be connected by a pipe, a hose, etc.

The steam generator 700 for the movement of steam and the inner cabinet 100 may be connected by a pipe, a hose, etc. In the shoes care device 1 according to one embodiment of the present invention, as described below, the steam generated by the steam generator 700 may be supplied into the inner cabinet 100 after passing through the steam valve 710 and a steam separator 720. In this case, in order to move the steam, the steam generator 700 and the steam valve 710 may be connected by a pipe, a hose, etc., and the steam valve 710 and the steam separator 720 may also be connected by a pipe, a hose, etc.

The steam valve 710 may be disposed adjacent to the steam generator 700, and the steam valve 710 may be provided in the machine room 50. The steam generator 700 and the steam valve 710 may be provided below the second management device 2b.

The steam valve 710 is configured to selectively communicate with each of the accommodation space 101 of the first management device 2a and the accommodation space 101 of the second management device 2b, respectively.

When the steam of the steam generator 700 is supplied to the accommodation space 101 of the first management device 2a and/or the accommodation space 101 of the second management device 2b, the steam valve 710 operates to control whether the stream is supplied or not, and an operation of the stream 710 is driven by the controller 10.

The steam valve 710 includes a valve housing 711, a valve inlet 712, a first valve outlet 713, a second valve outlet 714, a valve disk 715, and a valve motor 716.

In the steam valve 710 illustrated in FIGS. 7a and 7b, the other outlets except the valve inlet 712, the first valve outlet 713, and the second valve outlet 714 may be blocked by a stopper and deactivated.

The valve housing 711 forms a body of the steam valve 710, and has a predetermined inner space formed inside.

The valve inlet 712 may have a tubular shape and be coupled to the valve housing 711 to communicate with the inner space of the valve housing 711. The valve inlet 712 is a part connected to the steam generator 700, and steam may flow into the steam valve 710 (inside the valve housing 711) through the valve inlet 712.

The first valve outlet 713 and the second valve outlet 714 may be formed in a tubular shape and be coupled to the valve housing 711 to communicate with the inner space of the valve housing 711.

The first valve outlet 713 and the second valve outlet 714 are outlets through which steam is discharged from the steam valve 710. The first valve outlet 713 is connected to the accommodation space 101 of the first management device 2a, and the second valve outlet 714 is connected to the accommodation space 101 of the second management device 2b.

The valve disk 715 is provided inside the steam valve 710 (inside the valve housing 711) and is configured to open and close a flow path inside the steam valve 710. The valve disk 715 may be configured to selectively open and close a flow path of the first valve outlet 713 and a flow path of the second valve outlet 714.

The valve disk 715 is disposed between the valve inlet 712 and the first valve outlet 713, or between the valve inlet 712 and the second valve outlet 714, inside the valve housing 711. The valve disk 715 allows the valve inlet 712 and the first valve outlet 713 to communicate with each other or block the communication therewith, and also allows the valve inlet 712 and the second valve outlet 714 to communicate with each other or block the communication therewith.

In one embodiment of the present invention, the valve disk 715 may be formed in a circular plate shape and may include a valve hole 715a. The valve hole 715a is a hole penetrating the valve disk 715. The valve disk 715 may be rotatably coupled to the valve housing 711 around a valve rotation axis 715b, and the valve hole 715a may be formed eccentrically from the valve rotation axis 715b.

The valve motor 716 is coupled to the valve housing 711, and the valve motor 716 is coupled to the rotation axis 715b of the valve disk 715 to rotate the valve disk 715.

The controller 10 may control the steam valve 710 by controlling an operation of the valve motor 716.

The valve disk 715 rotates by the operation of the valve motor 716, and the valve disk 715 opens or closes a flow path inside the steam valve 710 (inside the valve housing 711) depending on the degree of rotation of the valve disk 715.

In one embodiment, depending on the degree of rotation of the valve disk 715, when the valve inlet 712 and the first valve outlet 713 communicate with each other through the valve hole 715a, the valve inlet 712 and the second valve outlet 714 are blocked from communicating with each other by the valve disk 715, or when the valve inlet 712 and the second valve outlet 714 communicate with each other through the valve hole 715a, the valve outlet 712 and the first valve outlet 713 may be blocked from communicating with each other by the valve disk 715.

In another embodiment, depending on the degree of rotation of the valve disk 715, the valve inlet 712 may communicate with both the first valve outlet 713 and the second valve outlet 714, or the valve inlet 712 may be blocked from communicating with both the first valve outlet 713 and the second valve outlet 714.

In the shoes care device 1 according to one embodiment of the present invention, the valve disk 715 may close a flow path of the second valve outlet 714 while opening a flow path of the first valve outlet 713, and may also close the flow path of the first valve outlet 713 and open the flow path of the second valve outlet 714.

The steam valve 710 made as described above may operate such that only the valve inlet 712 and the first valve outlet 713 communicate with each other, or only the valve inlet 712 and the second valve outlet 714 communicate with each other.

In the arrangement explained above, all the steam generated by the steam generator 700 may be supplied to the accommodation space 101 of the first management device 2a, or may be supplied to the accommodation space 101 of the second management device 2b. In this case, the steam generated by the steam generator 700 may be supplied to the accommodation space 101 of the first management device 2a or the accommodation space 101 of the second management device 2b without a pressure drop, and even when only one steam generator 700 is provided in the shoes care device 1, the steam may be sufficiently and stably supplied to the accommodation spaces 101 of each of the two inner cabinets 100.

In one embodiment of the present invention, the controller 10 may control the stream valve 710 such that when the heating part 320 of the first management device 2a is turned off (when a heater 321 of the heating part 320 is turned off), the valve disk 715 closes or opens the first valve outlet 713, and when the heating part 320 of the first management device 2a is turned on (when the heater 321 of the heating part 320 is turned on), the valve disk 715 closes the first valve outlet 713.

In addition, the controller 10 may control the stream valve 170 such that when the heating part 320 of the second management device 2b is turned off (when the heater 321 of the heating part 320 is turned off), the valve disk 715 closes or opens the second valve outlet 714, and when the heating part 320 of the second management device 2b is turned on (when the heater 321 of the heating part 320 is turned on), the valve disk 715 closes the second valve outlet 714.

In the shoes care device 1 according to one embodiment of the present invention, by controlling the steam valve 710 by the controller 10, the steam generated in the steam generator 700 may be selectively or simultaneously supplied to the accommodation space 101 of the first management device 2a and the accommodation space 101 of the second management device 2b, and whether the steam is supplied or not may be controlled according to a use state of the shoes care device 1.

FIG. 8a is a cross-sectional view taken along line D-D' of the shoes care device 1 illustrated in FIG. 3.

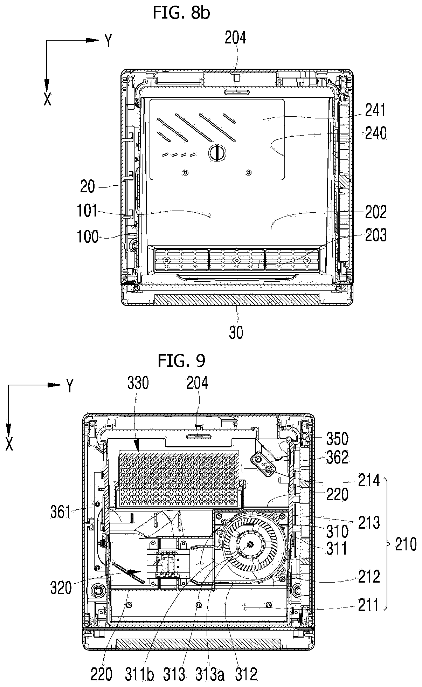

FIG. 8b is a view illustrating a state in which the main shelf 40 is removed from the shoes care device 1 illustrated in FIG. 8a.

FIG. 9 is a cross-sectional view taken along line E-E' of the shoes care device 1illustrated in FIG. 3.

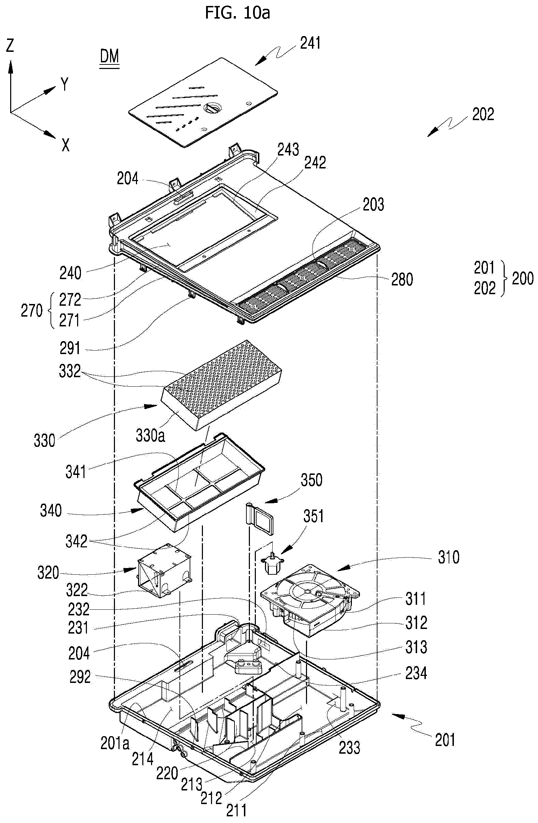

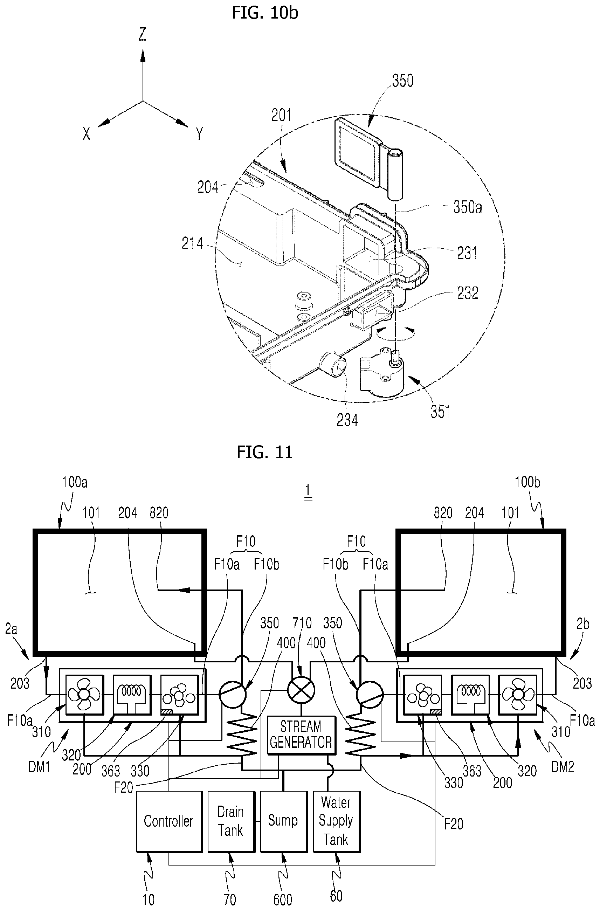

FIG. 10a is an exploded perspective view illustrating a drying module in the shoes care device 1 according to one embodiment of the present invention. FIG. 10b is an exploded perspective view illustrating a partial configuration of the drying module at a part to which the damper 350 is coupled.

In the shoes care device 1 according to one embodiment of the present invention, the dehumidifying part 330 may be used as a means that dehumidifies air.

As described above, the dehumidifying part 330 may be provided inside the module housing 200.

The dehumidifying part 330 is configured to have a predetermined volume. The dehumidifying part 330 may be configured to be porous by itself. A plurality of pores may be formed over an entire volume of the dehumidifying part 330, and air may move by penetrating the dehumidifying part 330 through such pores.

When the dehumidifying part 330 is composed of a combination of a plurality of dehumidifying materials, the plurality of dehumidifying materials may be fixed to each other by a separate fixing means, or may be fixed to each other by adhesion.

The dehumidifying part 330 may be formed of dehumidifying materials, and may be configured to include the dehumidifying materials.

The dehumidifying material 331 according to one embodiment of the present invention is configured to include a material capable of reducing humidity by absorbing moisture in the air. The dehumidifying material 331 may be formed of various materials or a combination of the materials within the range of absorbing or adhering the moisture in the air, and may be formed in various shapes and structures.

The dehumidifying material 331 according to one embodiment of the present invention may be referred to as a desiccant or an adsorbent.

The dehumidifying material 331 according to one embodiment of the present invention may be formed of a microporous material. The dehumidifying material 331 according to one embodiment of the present invention may include silica gel, activated carbon, activated alumina (AL2O3), diatomaceous earth, etc.

Specifically, the dehumidifying material 331 according to one embodiment of the present invention may be formed of zeolite or may be configured to include zeolite.

The zeolite is a natural and synthetic silicate mineral in which tunnels or open channels having a size of approximately 3 to 10 angstroms (A) are regularly arranged, and may function as dehumidification by adsorbing the moisture in the air.

When the zeolite is heated, moisture adsorbed on the zeolite may be separated into a large amount of steam. According to the characteristics of the zeolite, the zeolite may be regenerated in a state capable of not only performing the dehumidification function to remove the moisture from the air but also performing the dehumidification function by heating the zeolite and separating the moisture adsorbed to the zeolite.

The zeolite may be formed in the form of small grains (or stones) having the size (diameter) of several micrometers to several tens of micrometers, and the dehumidifying material 331 described in one embodiment of the present invention may refer to a combination of the grains (or stones). Each of the grains (or stones) may be agglomerated or combined with each other to form a single structure.

In another embodiment, the dehumidifying part 330 may include a dehumidifying body 330a and the dehumidifying material 331.

The dehumidifying body 330a may be formed to have a predetermined volume. In one embodiment, the dehumidifying body 330a may be formed in a substantially hexahedral shape.

In order to allow air to move through the dehumidifying body 330a, the dehumidifying body 330a may be provided with a plurality of dehumidification through holes 332 penetrated in one direction. A cross section of the dehumidifying through hole 332 may be formed in a circular shape, a polygonal shape, etc. The dehumidifying through hole 332 may have a hexagonal cross section.

In the dehumidifying body 330a, the dehumidifying through hole 332 may all have the same shape and size, or may have different shapes and sizes.

The dehumidifying body 330a may be formed of or include materials such as synthetic resin, metal, ceramic, etc. The dehumidifying body 330a may be formed of a combination of fibers, and may be formed of a nonwoven fabric, etc.

The dehumidifying material 331 may be coated on the dehumidifying body 330a. The dehumidifying material 331 may be coated on the outside and inside of the dehumidifying body 330a. Specifically, the dehumidifying material 331 may be coated on a surface in which the dehumidifying through hole 332 is formed.

When the dehumidifying body 330a is formed of a combination of fibers, the zeolite may be first coated on each fiber as the dehumidifying material 331, and the zeolite-coated fiber may be processed to form the dehumidifying body 330a and simultaneously form the dehumidifying part 330.

Regarding the coating of the zeolite, a manufacturing method of a zeolite coated ceramic paper (Korean Patent No. 10-1004826) is known, and Korean Patent No. 10-1173213 and Korean Patent No. 10-0941521 also describes a method of coating zeolite on a surface of a material. The dehumidifying part 330 according to one embodiment of the present invention may be formed by coating the gelled zeolite precursor on the dehumidifying body 330a or materials constituting the dehumidifying body 330a and then performing heat treatment when the dehumidifying material 331 is formed of the zeolite.

In one embodiment of the present invention, the coating of the dehumidifying material 331 (zeolite) may be performed by using various known or possible methods, and is not limited to a certain manufacturing method related to the coating of the dehumidifying material 331.

The blowing part 310 is provided inside the connection path F10. A blowing fan 313 may be provided inside the blowing part 310. Since the blowing part 310 is provided in the connection path F10, air flows in the connection path F10 when the blowing part 310 is driven, and since the connection path F10 also communicates with the accommodation space 101 of the inner cabinet 100, the air flows and moves in the accommodation space 101 by driving the blowing part 310.

In this way, the air may be sucked from the inner cabinet 100 into the connection path F10 by the driving of the blowing part 310 (a rotation of the blowing fan 313), and the air inside the connection path F10 may be ventilated.

In one embodiment, the blowing part 310 is provided inside the module housing 200 forming the connection path F10, and the blowing part 310 is driven to suction the air inside the inner cabinet 100 into an inlet 203. The air inside the connection path F10 passes through the module housing 200 constituting the connection path F10, the dry air duct 370, and a nozzle duct 810 and is then discharged back into the inner cabinet 820.

As such, the flow of air may be generated in the shoes care device 1 by the driving of the blowing part 310.

Dry air may be supplied to the inside of the inner cabinet 100 by the blowing part 310.

The heating part 320 is provided at one side of the dehumidifying part 330 and the blowing part 310 in the connection path F10. The heating part 320 may be provided inside the module housing 200. Based on a movement direction of the air inside the module housing 200, the module housing 200 may be arranged in an order of the blowing part 310, the heating part 320, and the dehumidifying part 330. That is, the air introduced into the outlet 203 of the module housing 200 moves along the connection path F10 by passing sequentially through the blowing part 310, the heating part 320, and the dehumidifying part 330.

The heating part 320 is disposed inside the module housing 200 and is configured to heat the air of the module chamber 210 inside the module housing 200.

The heating part 320 may be configured to heat the dehumidifying part 330. The heating part 320 may be configured to heat the dehumidifying material 331 constituting the dehumidifying part 330.

The air heated by the heating part 320 by the driving of the blowing part 310 moves directly to the dehumidifying part 330, thus heating the dehumidifying part 330. To this end, the heating part 320 is disposed inside the module housing 200 adjacent to the dehumidifying part 330. Specifically, the heating part 320 is disposed inside the module housing 200 adjacent to the dehumidifying part 330 based on a movement path of the air inside the module housing 200.

In the module housing 200, the dehumidification by the dehumidifying material 331 or the regeneration of the dehumidifying material 331 may be achieved by selectively heating the heating part 320.

The heating part 320 may be fixedly coupled to the module housing 200 in the module housing 200.

The heating part 320 may be made up of various devices and structures within a range capable of heating the air inside the module housing 200 or supplying heat to the dehumidifying part 330.

The heating part 320 may be formed of an electric heater 321. In one embodiment of the present invention, the heating part 320 may include the heater 321. The heater 321 includes a heating element, and may be configured to supply heat to the periphery while the heating element generates heat by supplied electric energy. The heater 321 may include a nichrome wire as the heating element.

The heater 321 of the heating part 320 may be formed in a ring shape, and the air may move by penetrating the center and surroundings of the ring-shaped heater 321 and be simultaneously heated. The heater 321 of the heating part 320 may be repeatedly formed in a second module chamber 213 along the movement direction of air.

The heater 321 of the heating part 320 may be formed in a circular ring shape or a rectangular ring shape.

The heating part 320 may include a heater flange 322 to which the heater 321 is fixed.

The heater flange 322 may be formed in the form of a metallic plate.

The heater flange 322 may be formed of a combination of flat plates in the second module chamber 213 along the movement direction of air. The heater flange 322 has a cross section that may be formed of a plate shape or a combination of plates in the second module chamber 213 along the movement direction of air (the second direction (Y direction)).

The heater flange 322 may include an outer flange 322a and an inner flange 322b.

The outer flange 322a may be formed in a tubular shape along the second direction (Y direction). An interior of the outer flange 322a is provided with a space to move air along the movement direction of air (a direction parallel to the second direction (Y direction)) in the second module chamber 213.

The inner flange 322b is fixed to the interior of the outer flange 322a. The inner flange 322b may include two or more plates crossing each other, and the heater 321 of the heating part 320 may be fixed to the inner flange 322b.

The heater flange 322 may be formed in various forms that fix the heater 321 of the heating part 320 and do not interfere with a flow of air moving through the second module chamber 213.

In one embodiment of the present invention, the blowing part 310, the heating part 320, the dehumidifying part 330, and the module housing 200 may form one set.

The set may be provided in a plural form. The shoes care device 1 according to one embodiment may be provided with two sets.

Such a set may be provided in each of the first management device 2a and the second management device 2b.

Such a set may form a drying module DM in the shoes care device 1 according to one embodiment of the present invention.

That is, in one embodiment of the present invention, the drying module DM may include the module housing 200, the blowing part 310, the heating part 320, and the dehumidifying part 330. Furthermore, the drying module DM is provided in each of the first management device 2a and the second management device 2b.

In the shoes care device 1 according to one embodiment of the present invention, a plurality of drying modules DM may be provided.

In the shoes care device 1 according to one embodiment of the present invention, a pair of drying modules DM may be provided. When the shoes care device 1 is provided with the pair of drying modules DM, one of the drying modules DM may form a 'drying module A (DM1) ' as a drying module of the first management device 2a, and the other may form a 'drying module B (DM2) ' as a drying module of the second management device 2b.

The 'drying module' described in one embodiment of the present invention may be understood to refer to each of the 'drying module A' and the 'drying module B' except as otherwise particularly limited.

In the shoes care device 1 according to one embodiment of the present invention, the drying module A (DM1) and the drying module B (DM2) may operate in different modes. When the drying module A DM1 operates in a moisture absorption mode, the drying module B DM2 may operate in a regeneration mode. Conversely, when the drying module A DM1 operates in the regeneration mode, the drying module B DM2 may operates in the moisture absorption mode.

The 'moisture absorption mode' described in the present invention means a case in which the dehumidifying part 330 adsorbs moisture in the air, and the 'regeneration mode' means a case in which the moisture adsorbed to the dehumidifying part 330 is separated by heating the dehumidifying part 330.

Naturally, both the drying module A DM1 and the drying module B DM2 may operate in the moisture absorption mode or may operate in the regeneration mode.

The module housing 200 may be fixedly coupled to a lower side of the inner cabinet 100. The module housing 200 may be detachably coupled to a lower side of the inner cabinet 100.

The module housing 200 includes the module chamber 210 that is a space having other components accommodated inside. That is, the module chamber 210 is a space inside the module housing 200 distinguished from an external space of the module housing 200. As described above, the module housing 200 forms part of the connection path F10, and accordingly, the module chamber 210 is configured to communicate with a space outside the module housing 200. The module chamber 210 communicates with the accommodation space 101 of the inner cabinet 100.

The module housing 200 may include a module case 201 and a module cover 202.

The module case 201 and the module cover 202 may be formed by injection molding, respectively, and may be assembled with each other after manufacturing to form the module housing 200.

The module case 201 is formed in the form of a container that is concave substantially downwards, and forms the module chamber 210 of the module housing 200.

The module case 201 may be configured in the form of a container opened upwards, and includes a module opening 201a.

In a plan view, an area of the module opening 201a may be larger than or equal to an area of the module chamber 210.

The module chamber 210 may include a first module chamber 212, a second module chamber 213, and a third module chamber 214. The module chamber 210 may include a suction module chamber 211.

In order to distinguish between the suction module chamber 211, the first module chamber 212, the second module chamber 213, and the third module chamber 214, a module partition wall 220 may be formed inside the module housing 200. Furthermore, the module partition wall 220 guides the movement of air so that air moves in a predetermined direction inside the module housing 200.

The suction module chamber 211 is a first space where air is introduced into the module housing 200.

The first module chamber 212 is a space in which the blowing part 310 is accommodated, the second module chamber 213 is a space in which the heating part 320 is accommodated, and the third module chamber 214 is a space in which the dehumidifying part 330 is accommodated.

In one embodiment of the present invention, the suction module chamber 211, the first module chamber 212, the second module chamber 213, and the third module chamber 214 may be formed at different positions in a plan view.

In addition, the air of the module chamber 210 may be configured to move the suction module chamber 211, the first module chamber 212, the second module chamber 213, and the third module chamber 214 sequentially. That is, when the blowing part 310 is driven, air moves sequentially through the suction module chamber 211, the first module chamber 212, the second module chamber 213, and the third module chamber 214 inside the module housing 200.

The module case 201 may include a dry air outlet 231 and a wet air outlet 232.

The dry air outlet 231 may be formed in a hole shape opened to allow air in the third module chamber 214 to flow out. The dry air outlet 231 is formed adjacent to the third module chamber 214. The dry air outlet 231 may be formed on one edge of the module case 201. Furthermore, the dry air outlet 231 may be connected to the accommodation space 101 through the dry air duct 370 and the nozzle duct 810.

The wet air outlet 232 may be formed in a hole shape opened to allow the air in the third module chamber 214 to flow out. The wet air outlet 232 is formed adjacent to the third module chamber 214. The wet air outlet 232 may be formed on a frame on one side of the module case 201. Furthermore, the wet air outlet 232 may be connected to the condenser 400.

The dry air outlet 231 and the wet air outlet 232 may be formed adjacent to each other. The dry air outlet 231 and the wet air outlet 232 may be formed adjacent to any one vertex part of the module housing 200.

The suction module chamber 211 is formed adjacent to the first module chamber 212, and a bottom surface of the suction module chamber 211 may be inclined downwardly toward the first module chamber 212. Accordingly, air introduced into the suction module chamber 211 may naturally move toward the first module chamber 212 by hitting the bottom surface of the suction module chamber 211 forming an inclined surface, and a condensed water introduced into the suction module chamber 211 may move along the bottom surface of the suction module chamber 211 forming the inclined surface, and may move to the first module chamber 212.

The blowing part 310 may be assembled to the module housing 200 while being spaced apart from a bottom surface of the first module chamber 212. Furthermore, in this case, the blowing part 310 may be configured such that air may be introduced from a lower side of the first module chamber 212 to an interior of the blowing part 310 inside the first module chamber 212.

The module case 201 may include a first condensed water discharge hole 233.

The first condensed water discharge hole 233 is formed in a hole shape penetrating the module case 201. The first condensed water discharge hole 233 is formed on an edge of the module case 201 adjacent to a condenser 400 and formed to be equal to or lower than the bottom surface of the first module chamber 212, and communicates with the condenser 400. Among the bottom surfaces of the first module chamber 212, the first condensed water discharge hole 233 may form the lowest part, or the bottom surface of the first module chamber 212 may be formed such that a height thereof is lowered toward or at least equal to the first condensed water discharge hole 233.

As such, the first condensed water discharge hole 233 may be lower than the bottom surface of the first module chamber 212, and accordingly, the condensed water introduced into the first condensed water discharge hole 232 may move toward the first condensed water discharge hole 233 and flow into the condenser 400 through the first condensed water discharge hole 233.

Meanwhile, since the first condensed water discharge hole 233 is a hole in which the module housing 200 and the condenser 400 communicate with each other, the air inside the condenser 400 may flow into the module housing 200 through the first condensed water discharge hole 233. The air introduced into the module housing 200 through the first condensed water discharge hole 233 from an interior of the condenser 400 may move along the first module chamber 212, the second module chamber 213, and the third module chamber 214 by an operation of the blowing part 310 and may be introduced again into the condenser 400 and condensed.

The shoes care device 1 according to one embodiment of the present invention includes the condenser 400 coupled to an outer surface of the inner cabinet 100 and forming a regeneration path F20. In a plan view, the first module chamber 212 may be provided between the second module chamber 213 and the condenser 400. Since the first module chamber 212 is disposed between the second module chamber 213 and the condenser 400, a direct heat exchange between the condenser 400 and the heating part 320 is blocked, and the heat may be prevented from being transferred to the condenser 400 when the heating part 320 is heated inside the second module chamber 213.

Accordingly, when the dehumidifying part 330 is regenerated, condensation depending on heating of air by the heater 321 of the heating part 320 and cooling of air inside the condenser 400 may be effective performed.

The dehumidifying part 330 may be coupled to the module housing 200 while being spaced apart from a bottom surface of the third module chamber 214. Furthermore, in this case, the air inside the third module chamber 214 may move downwards through the dehumidifying part 330 from an upper side of the third module chamber 214.

The module case 201 may include a second condensed water discharge hole 234.

The second condensed water discharge hole 234 is formed in a hole shape penetrating the module case 201. The second condensed water discharge hole 234 is formed on the edge of the module case 201 adjacent to the condenser 400 and formed to be equal to or lower than the bottom surface of the third module chamber 214, and communicates with the condenser 400. Among the bottom surfaces of the third module chamber 214, the second condensed water discharge hole 234 may form the lowest part, or the bottom surface of the third module chamber 214 may be formed such that a height thereof is lowered toward or at least equal to the second condensed water discharge hole 234.

The second condensed water discharge hole 234 may be formed adjacent to the wet air outlet 232.

In this way, the second condensed water discharge hole 234 may be lower than the bottom surface of the third module chamber 214, and accordingly, condensed water introduced into the third module chamber 214 may move toward the second condensed water discharge hole 234, and may flow into the condenser 400 through the second condensed water discharge hole 234.

Meanwhile, since the second condensed water discharge hole 234 is a hole in which the module housing 200 and the condenser 400 communicate with each other, the air inside the condenser 400 may flow into the module housing 200 through the second condensed water discharge hole 234. In this way, the air introduced from the interior of the condenser 400 into the module housing 200 through the second condensed water discharge hole 234 moves directly to the wet air outlet 232 by the driving of the blowing part 310, and may be introduced again into the condenser 400 and condensed.

The module housing 200 may include the module cover 202.

The module cover 202 is coupled to the module case 201 while shielding the module opening 201a from an upper side of the module case 201. The module cover 202 may be detachably coupled to the module case 201. A plurality of locking projections 292 may protrude from one of the module cover 202 and the module case 201, and a plurality of locking grooves 291 into which the locking projections 292 are inserted and locked may be formed on the other. The locking projections 292 and locking grooves 291 are provided in a plural form, respectively, and may be spaced apart along an edge of the module housing 200 and repeatedly formed.

With the blowing part 310, the heating part 320, and the dehumidifying part 330 accommodated in the module case 201, the module cover 202 may shield the blowing part 310, the heating part 320, and the dehumidifying part 330 and may be coupled to the module case 201.

The shoes care device according to one embodiment of the present invention may be formed in a structure in which the dehumidifying part 330 may be detachable from the module housing 200. The structure of the shoes care device provides an advantageous advantage in maintaining and managing the dehumidifying part 330 and the shoes care device 1 on the whole.

On the other hand, the dehumidifying part 330 may be repeatedly used by regeneration, but with the repeated use, the dehumidifying part 330 needs to be replaced.

Considering these descriptions, the shoes care device 1 according to one specific embodiment of the present invention may be configured to separate and replace the dehumidifying part 330.

In one embodiment of the present invention, the module cover 202 of the module housing 200 may form a bottom surface of the inner cabinet 100.

The module cover 202 may form a boundary surface between the inner cabinet 100 and the module housing 200. The module cover 202 may be formed in a substantially rectangular shape.

The module cover 202 may be configured in substantially parallel with the horizontal direction.

Alternatively, the module cover 202 may be inclined to any one side. In one embodiment, an upper surface of the module cover 202 may be inclined downwardly toward the first direction (X direction) (a front side of the shoes care device 1).

In one embodiment of the present invention, the main shelf 40 is mounted in close contact with an upper side surface of the module cover 202, and the main shelf 40 mounted on the upper side surface of the module cover 202 is also configured to be inclined when the upper side surface of the module cover 202 is inclined. In this case, since an upper surface of the main shelf 40 is inclined, water (e.g., condensed water) placed on the upper surface of the main shelf 40 may flow along an inclined direction.

The shoes care device 1 may include a dehumidifying material cover 241.

The dehumidifying material cover 241 forms part of the module cover 202 that is the bottom of the inner cabinet 100. Furthermore, the dehumidifying material cover 241 may be detached from the module cover 202 of the inner cabinet 100 or may be hinge-coupled to the module cover 202.

In the module cover 202, a dehumidifying material exit 240 which is an opening of a shape and a size corresponding to the dehumidifying material cover 241 may be formed. The dehumidifying material cover 241 may be configured to open and close the dehumidifying material exit 240. The dehumidifying material cover 241 may be tightly coupled to the dehumidifying material exit 240. At least a part of the dehumidifying material cover 241 may be separated from the module cover 202. In one embodiment, the dehumidifying material exit 240 of the module cover 202 may be opened while completely separating the dehumidifying material cover 241 from the module cover 202, and in another embodiment, the dehumidifying material cover 241 of the module cover 202 may be opened while rotating the dehumidifying material cover 241 around a hinge axis. The dehumidifying part 330 may be introduced into or withdrawn from the module housing 200 through the dehumidifying material exit 240.

The dehumidifying material exit 240 and the dehumidifying material cover 241 may be formed in a position corresponding to the third module chamber 214 in a plan view. That is, the dehumidifying material exit 240 and the dehumidifying material cover 241 may be formed directly above the third module chamber 214. The shoes care device 1 according to one embodiment of the present invention may be configured such that the first module chamber 212 and the second module chamber 213 are not exposed in a plan view in a state where the dehumidifier cover 241 is opened.

When the dehumidifying material cover 241 is opened in the module cover 202, the third module chamber 214 disposed on a lower part of the module cover 202 is exposed through the dehumidifying material exit 240 of the module cover 202, and the dehumidifying part 330 may be settled inside the module case 201, or may be immediately withdrawn and separated from the module case 201.

The sizes and shapes of the dehumidifying material cover 241 and the dehumidifying material exit 240 are variously provided within the range capable of withdrawing or inserting the dehumidifying part 330.

The dehumidifying material cover 241 may be formed in a rectangular plate shape.

The length of the dehumidifying material cover 241 in the first direction (X direction) may be equal to or longer than the length of the dehumidifying part 330, and the length of the dehumidifying material cover 241 in the second direction (Y direction) may be equal to or longer than the length of the dehumidifying part 330.

As described above, in the shoes care device 1 according to one embodiment of the present invention, the heating part 320 and the dehumidifying part 330 are formed at different positions in a top plane view, and when the dehumidifying material cover 241 is opened on the module cover 202 that forms the bottom surface of the inner cabinet 100, the dehumidifying part 330 disposed directly below the dehumidifying material cover 241 may be withdrawn from the module housing 200, and the dehumidifying part 330 may be easily replaced by the user.

In addition, since only the third module chamber 214 is exposed in a state in which the dehumidifier cover 241 is opened, and the first module chamber 212 and the second module chamber 213 are not exposed, the blowing part 310 accommodated in the first module chamber 212 and the heating part 320 accommodated in the second module chamber 213 are not exposed. That is, since the blowing part 310 and the heating part 320 are not directly exposed to the user, safety accidents due to an unintended operation of the blowing part 310 and/or the heating part 320 can be prevented.

The dehumidifying material cover 241 may be configured to separately shield the dehumidifying part 330. A space between the dehumidifying material cover 241 and the dehumidifying part 330 may form a part of the connection path F10.

As described above, the outlet 203 forms an inlet through which the air inside the inner cabinet 100 is sucked into the module housing 200. The outlet 203 may form a start part of the connection path F10. The outlet 203 may be formed in the shape of a hole vertically penetrated from the bottom surface (the upper surface of the module cover 202) of the inner cabinet 100.

A network such as a grid shape, a mesh shape, etc., may be formed on the outlet 203.

The outlet 203 may be formed parallel to the second direction (Y direction). That is, the outlet 203 may be formed in a long hole shape in the module cover 202 along the second direction (Y direction).

The outlet 203 may be formed on an edge of the module cover 202. The outlet 203 may be formed on the edge of the module cover 202 along the second direction (Y direction).

The outlet 203 may be formed on a front part or a rear part of the module cover 202 based on the first direction (X direction).

The outlet 203 may be disposed relatively close to the door 30 in the module cover 202. That is, the outlet 203 may be disposed relatively in the front of the module cover 202.

The upper surface of the module cover 202 may be inclined downwardly toward the outlet 203. That is, a part of the module cover 202 where the outlet 203 is formed may be configured to be the lowest. Accordingly, when water is present on the module cover 202 or the main shelf 40, such water may flow along the surface of the module cover 202 by gravity and flow into the inlet 203.

In the shoes care device 1 according to one embodiment of the present invention, the module chamber 210 is provided inside the module housing 200, and the module chamber 210 includes a first module chamber 212, a second module chamber 213, and the third module chamber 214. The first module chamber 212, the second module chamber 213, and the third module chamber 214 may be formed at different positions in a plan view. That is, the blowing part 310, the heating part 320, and the dehumidifying part 330 may be disposed at different positions in the module housing 200. According to one embodiment of the present invention, the blowing part 310, the heating part 320, and the dehumidifying part 330, which are main means for drying the air inside the inner cabinet 100 and main means for regenerating the dehumidifying part 330, are disposed together in the module chamber 210 of the module housing 200. Accordingly, the blowing part 310, the heating part 320, and the dehumidifying part 330 are disposed at positions considerably close to each other.

In one embodiment of the present invention, the module case 201 of the module housing 200 may be integrally formed by injection molding. In this case, the bottom parts of the module housing 200 may be integrally formed, the bottom parts may not be assembled with each other, and no gaps may be formed in the bottom parts.

In the arrangement explained above, the condensed water can be effectively prevented from leaking from the module housing 200. In addition, a vertical height of the module housing 200 can be minimized.

When moisture remains at an unintended part inside the shoes care device 1, such moisture may reproduce bacteria or cause odors. This is why there is need for countermeasures to solve the problems, and the shoes care device 1 according to one embodiment of the present invention can effectively prevent water from leaking in consideration of such problems.

In the shoes care device 1 according to one embodiment of the present invention, the air in the module chamber 210 may sequentially move the first module chamber 212, the second module chamber 213, and the third module chamber 214. Accordingly, since the third module chamber 214 and a drying flow path F10b may be connected in the shortest distance, which provides excellent drying efficiency by the dehumidifying part 330, and air heated by the heating part 320 moves directly to the dehumidifying part 330 to form the shoes care device 1 with excellent regeneration efficiency.

In the shoes care device 1 according to one embodiment of the present invention, the module housing 200 includes the suction module chamber 211, and the bottom surface of the suction module chamber 211 may be inclined downwardly toward the first module chamber 212. Accordingly, the air introduced through the outlet 203 moves naturally to the first module chamber 212 by hitting the bottom surface of the suction module chamber 211, and the condensed water introduced into the outlet 203 moves to the first module chamber 212 such that the condensed water can be easily drained.

The shoes care device 1 according to one embodiment of the present invention may include the condenser 400, and the module case 201 may include the first condensed water discharge hole 233. In addition, the module case 201 may include the second condensed water discharge hole 234. Accordingly, the dehumidifying part 330 may be effectively regenerated, and condensed water inside the module housing 200 may be easily discharged to the condenser 400.

In the shoes care device 1 according to one embodiment of the present invention, steam generated by the steam generator 700 is supplied to the accommodation space 101 of the inner cabinet 100, and to this end, the shoes care device 1 includes a steam inlet 204.

The steam inlet 204 forms an inlet through which steam is supplied to the accommodation space 101 of the inner cabinet 100.

In the shoes care device according to one embodiment of the present invention, the steam inlet 204 is formed in the module housing 200.

The steam inlet 204 may be formed in a rear part of the module housing 200 based on the first direction (X direction). The steam inlet 204 may be formed to vertically penetrate the module housing 200. The steam inlet 204 may be formed to vertically penetrate the module case 201 and the module cover 202. The steam inlet 204 may be formed in the center in a right-left direction at the rear part of the module housing 200.

The steam inlet 204 may be formed on a rear edge of the module housing 200, and may be formed directly behind a position where the third module chamber 214 is formed. The third module chamber 214 and the steam inlet 204 are shielded from each other.

In the module housing 200, the module cover 202 forms the bottom surface of the accommodation space 101, and when the module housing 200 is coupled to the inner cabinet 100, the steam inlet 204 is formed behind the bottom of the inner cabinet 100.

The steam generator 700 and the steam valve 710 are disposed in a lower part, the distance from the steam generator 710 to the steam inlet 204 can be reduced by forming the module housing 200, and the steam inlet 204 in a rear part of the module housing 200, and an increase in the load required for the supply of steam can be prevented. Accordingly, steam may be smoothly supplied from the steam generator 700 to the steam inlet 204

FIG. 11 is a diagram illustrating a connection relationship between components and a flow of fluid in the shoes care device 1 according to one embodiment of the present invention.

The connection path F10 forms a movement path of air connected from the outlet 203 to the nozzle 820. That is, the outlet 203 may form an inlet of the connection path F10, and the nozzle 820 may form an outlet of the connection path F10.

The outlet 203 may be coupled to communicate with the inner cabinet 100, and the nozzle 820 may be provided inside the inner cabinet 100. Except the outlet 203 and the nozzle 820, one part of the connection path F10 may be provided inside the inner cabinet 100, and the other part may be provided outside the inner cabinet 100.

The air inside the inner cabinet 100 moves to the connection path F10 through the outlet 203, and the air passing through the connection path F10 moves back into the inner cabinet 100 through the nozzle 820. As such air flow is repeated, the air circulation is performed in the shoes care device 1.

In the nozzle 820, a hole through which air is discharged is formed in the accommodation space 101 of the inner cabinet 100, and the nozzle 820 may form a last part of the connection path F10.

In the shoes care device 1 according to one embodiment of the present invention, since the nozzle 820 is configured to be movable to various positions inside the inner cabinet 100, the shoes may be managed in various positions.

As described above, the dehumidifying part 330 is disposed in the connection path F10. The air moving through the connection path F10 passes through the dehumidifying part 330, and the dehumidifying part 330 absorbs moisture from the air moving through the connection path F10 such that the air from which moisture has been removed may be supplied into the inner cabinet 100.

The connection path F10 may be divided into a conversion flow path F10a and a drying flow path F10b. The conversion flow path F10a and the drying flow path F10b form a movement path of air sequentially connected to each other. The air in the connection path F10 may sequentially move through the conversion flow path F10a and the drying flow path F10b.

The conversion flow path F10a forms an upstream section of the connection path F10, which is connected to the outlet 203. The conversion flow path F10a may be a section in which the blowing part 310, the heating part 320, and the dehumidifying part 330 are disposed. The conversion flow path F10a may be formed by the module housing 200, and the module chamber 210 inside the module housing 200 may form the conversion flow path F10a.

The conversion flow path F10a may be a section in which humid air moves and dries. The conversion flow path F10a may be a section in which air is dehumidified by the dehumidifying part 330.

Meanwhile, the conversion flow path F10a may be a section in which the dehumidifying part 330 (the dehumidifying material 331) are regenerated.

The drying flow path F10b forms a downstream section of the connection path F10, which connects the conversion flow path F10a to the nozzle 820. A flow path formed by the drying air duct 370, the nozzle duct 810, and the nozzle 820 may form the drying flow path F10b.

The drying passage F10b may be a section in which dry air with moisture removed therefrom moves.

When the drying module DM operates in the moisture absorption mode, the drying flow path F10b communicates with the conversion flow path F10a, and when the drying module DM operates in the regeneration mode, the drying flow path F10b and the conversion flow path F10a may not communicate with each other such that the drying flow path F10b and the conversion flow path F10a block each other.

Accordingly, when air is dehumidified by the dehumidifying part 330 in the conversion flow path F10a, the dried air moves through the drying flow path F10b.

The dry air duct 370 may be fixedly coupled to an outer wall surface of the inner cabinet 100, and the nozzle duct 810 may be provided inside the inner cabinet 100.

As the dry air duct 370 is tightly coupled to an inner rear plate 110 of the inner cabinet 100, a flow path may be formed between the dry air duct 370 and the inner cabinet 100 (the inner rear plate 110), and such a flow path may form a part of the drying flow path F10b. A lower part of the dry air duct 370 communicates with the dry air outlet 231 of the module housing 200, an upper part thereof communicates with the nozzle duct 810, which connect the interior of the module housing 200 and the interior of the nozzle duct 810 for mutual communication.

As described above, after humid air in the accommodation space 101 of the inner cabinet 100 flows into the conversion flow path F10a, the air is dehumidified by the dehumidifying part 330 and converted into dry air, and the dry air can be resupplied to the accommodation space 101 of the inner cabinet 100 through the drying flow path F10b.

The regeneration path F20 forms a movement path of a fluid.

The regeneration path F20 forms a passage through which air and/or condensed water inside the shoes care device moves.

The regeneration path F20 forms a path through which air and/or condensed water passing through the dehumidifying part 330 moves when the dehumidifying material 331 is regenerated. The regeneration path F20 may be entirely or partially formed of a pipe, a hose, a tube, a duct, a housing, or a combination thereof.

Moisture generated during the regeneration process of the dehumidifying material 331 needs to be discharged through a separate flow path separated from the drying flow path F10b, which is a flow path through which dry air moves. Accordingly, the shoes care device 1 according to one embodiment of the present invention includes the regeneration path F20, and when the dehumidifying material 331 is regenerated, air passing through the dehumidifying part 330 is not ventilated to the nozzle 820 but moves through the regeneration path F20.

The regeneration path F20 is a flow path branched from the connection path F10. The regeneration path F20 may be branched from the connection path F10 to form a path different from that of the drying flow path F10b of the connection path F10. The regeneration path F20 is connected to the sump 600.

The regeneration path F20 may be a section that connects the conversion flow path F10a and the sump 600.

The regeneration path F20 may be a section in which humid air separated from the dehumidifying part 330 moves.

The condenser 400 according to one embodiment of the present invention forms a regeneration path F20. Moisture separated from the dehumidifying material 331 may be condensed after moving to the condenser 400 along with air moving along the regeneration path F20. In addition, condensed water condensed in the condenser 400 may be moved to the sump 600 through the regeneration path F20, collected from a lower part of the sump 600, and then discharged to the drain tank 70, discharged to the outside, or pressed to the steam generator 700.

In the shoes care device 1 according to one embodiment of the present invention, when the drying module DM operates in the regeneration mode, the regeneration path F20 communicates with the conversion flow path F10a, and when the drying module DM operates in the moisture absorption mode, the regeneration path F20 and the conversion flow path F10 may not communicate with each other such that the regeneration path F20 and the conversion flow path F10 block each other.

Accordingly, when the dehumidifying part 330 is regenerated in the conversion flow path F10a, humid air containing moisture separated from the dehumidifying part 330 moves through the regeneration path F20.

In one embodiment of the present invention, the damper 350 may be formed in the form of a damper valve.

The damper 350 may be rotatably coupled to the module housing 200. The damper 350 may be coupled to the module housing 200 in a form accommodated in the module housing 200.

As described above, in the module housing 200, the dry air outlet 231 forming an inlet of the drying flow path F10b is formed as a passage of the connection path F10, and the wet air outlet 232 forming an inlet of the regeneration path F20 is formed.

The damper 350 controls a movement path of air passing through the dehumidifying material 331 in the module housing 200. Depending on the operation of the damper 350, the air passing through the dehumidifying material 331 may move into the inner cabinet 100 through the nozzle 820 or may move into the regeneration path F20.

The damper 350 may be configured to open the regeneration path F20 while blocking the drying flow path F10b, or to open the drying flow path F10b while blocking the regeneration path F20.

The damper 350 may be configured to selectively shield the dry air outlet 231 and the wet air outlet 232. The damper 350 may be configured to selectively seal the dry air outlet 231 and the wet air outlet 232.

The damper 350 may selectively block one of the dry air outlet 231 and the wet air outlet 232. When the damper 350 opens the dry air outlet 231 while blocking the wet air outlet 232, the air passing through the dehumidifying material 331 may move into the inner cabinet 100 through the nozzle 820, and when the damper 350 opens the wet air outlet 232 while blocking the dry air outlet 231, the air passing through the dehumidifying material 331 may be condensed while moving through the regeneration path F20.

In the shoes care device 1 according to one embodiment of the present invention, the damper 350 may be configured to be hinge-rotatable around a hinge axis 350a formed on one side. The hinge axis 350a of the damper 350 may be parallel to the third direction (Z direction). In addition, the shoes care device 1 may include a damper motor 351 configured to rotate the damper 350 around the hinge axis 350a of the damper 350. The damper motor 351 may be formed as an electric motor and may be configured to rotate the damper 350 bidirectionally.

When the damper 350 opens the dry air outlet 231 and seals the wet air outlet 232, the air inside the inner cabinet 100 moves along the connection path F10 and is circulated by sequentially passing through the inlet 203, the module housing 200 (the blowing part 310 and the dehumidifying part 330, the dry air outlet 231, the dry air duct 370, the nozzle duct 810, and the nozzle 820.

When the damper 350 seals the dry air outlet 231 and opens the wet air outlet 232, the air moves along the conversion flow path F10a and the regeneration path F20 and is circulated by sequentially passing through the module housing 200 (the blowing part 310, the heating part 310, and the dehumidifying part 330), the wet air outlet 232, and the condenser 400.

In one embodiment of the present invention, the controller 10 may control the damper motor 351 such that the damper 350 closes the dry air outlet 231 and opens the wet air outlet 232 when the heating part 320 is turned on. In addition, the controller 10 may control the damper motor 351 such that the damper 350 opens the dry air outlet 231 and closes the wet air outlet 232 when the heating part 320 is turned off.

Accordingly, by controlling the damper motor 351 by the controller 10, the damper 350 may open the drying flow path F10b and close the regeneration path F20 when the heating part 320 is turned off, and may close the drying flow path F10b and open the regeneration path F20 when the heating part 320 is turned on.

The damper motor 351 may be controlled individually in each of the first management device 2a and the second management device 2b.

Referring to FIG. 11, in the drying module A (DM1) of the first management device 2a, the damper 350 seals the wet air outlet 232 and opens the dry air outlet 231 of the second management device 2b, and in the drying module B (DM2) of the second management device 2b, when the damper 350 opens the wet air outlet 232 and seals the dry air outlet 231, the air in the conversion flow path F10a of the first management device 2a may flow through the drying flow path F10b, and the air in the conversion flow path F10a of the second management device 2b may flow through the regeneration path F20. Furthermore, in this case, the drying module A (DM1) of the first management device 2a may operate in the moisture absorption mode, and the drying module B (DM2) of the second management device 2b may operate in the regeneration mode.

In contrast, when in the drying module A (DM1) of the first management device 2a, the damper 350 opens the wet air outlet 232 and seals the dry air outlet 231, and in the drying module B (DM2) of the second management device 2b, the damper 350 seals the wet air outlet 232 and opens the dry air outlet 231, the air in the conversion flow path F10a of the first management device 2a may flow along the regeneration path F20, and the air in the conversion flow path F10a of the second management device 2b may flow along the drying flow path F10b. Furthermore, in this case, the drying module A (DM1) of the first management device 2a may operate in the regeneration mode, and the drying module B (DM2) of the second management device 2b may operate in the moisture absorption mode.

In both the drying module A (DM1) of the first management device 2a and the drying module B (DM2) of the second management device 2b, when the damper 350 seals the wet air outlet 232 and opens the dry air outlet 231, both the drying module A DM1 and the drying module B (DM2) may operate in the moisture absorption mode.

In both the drying module A (DM1) of the first management device 2a and the drying module B (DM2) of the second management device 2b, when the damper 350 seals the dry air outlet 231 and opens the wet air outlet 232, both the drying module A (DM1) and the drying module B (DM2) may operate in the regeneration mode.

In the shoes care device 1 according to one embodiment of the present invention, the first management device 2a and the second management device 2b individually include the inner cabinet 100, the connection path F10, the blowing part 310, and the dehumidifying part 330. In addition, the shoes care device 1 includes the steam generator 700 and the steam valve 710. Accordingly, the degree of the supply of steam, the degree of dehumidification by the dehumidifying part 330, and the flow of air circulated along the connection path F10 may be different in each of the first management device 2a and the second management device 2b, and the first management device 2a and the second management device 2b may manage shoes under different conditions.

In addition, the first management device 2a and the second management device 2b include the module housing 200, the blowing part 310, the heating part 320, the dehumidifying part 330, and the drying flow path F10b, respectively. The air and condensed water moving in the first management device 2a and the air and condensed water moving in the second management device 2b move along different paths, thereby achieving accurate control intended in each of the first management device 2a and the second management device 2b. In this case, since the first management device 2a and the second management device 2b share and use the steam generator 700, the steam generator 700 can be efficiently utilized in the shoes care device 1, and the shoes care device 1 can efficiently utilize the space.

In addition, as described above, when the shoes are dried in one of the first management device 2a and the second management device 2b, the dehumidifying part 330 may be regenerated in the other, and the efficient management of the shoes and efficient use of the shoes care device 1 can be achieved.

In the shoes care device 1 according to one embodiment of the present invention, the first management device 2a and the second management device 2b each include the regeneration path F20 and the damper 350 individually.

The controller 10 may control the heating part 320 (the heater 321) and the damper 350 to interwork with each other.

The controller 10 may control the damper 350 to open the drying flow path F10b and close the regeneration path F20 when the heating part 320 is turned off, and may control the damper 350 to close the drying flow path F10b and open the regeneration path F20 when the heating part 320 is turned on.

The controller 10 may control each component of the shoes care device 1 such that air flowing into the module chamber 210 from the accommodation space 101 and passing through the dehumidifying part 330 moves along the drying flow path F10b when the heating part 320 is turned off (when the heater 321 of the heating part 320 is turned off), and the air moves along the regeneration path F20 when the heating part 320 is turned on (when the heater 321 of the heating part 320 is turned on).

Such control may be performed individually in each of the first management device 2a and the second management device 2b. Accordingly, since the movement path of air inside the module chamber 210 is changed depending on an operation of the heating part 320, the drying of the shoes and the regeneration of the dehumidifying part 330 can be effectively achieved.

The controller 10 may control the steam valve 710 and the heating part 320 to interwork with each other.

The controller 10 ma control the steam valve 710 such that when the heating part 320 of the first management device 2a is turned off, the valve disk 715 closes or opens the first valve outlet 713, when the heating part 320 of the first management device 2a is turned on, the valve disk 715 closes the first valve outlet 713, when the heating part 320 of the second management device 2b is turned off, the valve disk 715 closes or opens the second valve outlet 714, and when the heating part 320 of the second management device 2b is turned on, the valve disk 715 closes the second valve outlet 714.

In this way, the supply of steam to the accommodation space 101 of the inner cabinet 100 and the operation of the heating part 320 inside the module housing 200 may interwork with each other to effectively perform the drying of the shoes and the regeneration of the dehumidifying part 330.

The shoes care device 1 according to one embodiment of the present invention may include a first sensor 361 and a second sensor 362 (see FIG. 9).

The first sensor 361 may be installed in the second module chamber 213 of the module housing 200, and the second sensor 362 may be installed in the third module chamber 214 of the module housing 200. The first sensor 361 may be configured to measure the temperature and/or humidity of the second module chamber 213, and the second sensor 362 may measure the temperature and/or humidity of the third module chamber 214.

The first sensor 361 measures the temperature and/or humidity of air before passing through the dehumidifying part 330, and the second sensor 362 measures the temperature and/or humidity of air after passing through the dehumidifying part 330.

The controller 10 may compare the temperature and/or humidity of the second module chamber 213 measured by the first sensor 361 with the temperature and/or humidity of the third module chamber 214 measured by the second sensor 362 to recognize a state and a change of the temperature and/or humidity inside the module housing, and may also check an operation state of the drying module DM.

The controller 10 may recognize the temperature and humidity of the second module chamber 213 measured by the first sensor 361, and the temperature and humidity of the third module chamber 214 measured by the second sensor 362 to recognize a change in the humidity inside the module housing 200. Accordingly, the degree of dehumidification by the dehumidifying part 330 may be checked, and the degree of regeneration of the dehumidifying part 330 may be checked.

In one embodiment, when the drying module DM operates in the moisture absorption mode, the controller 10 may control the drying module DM to stop operating in the moisture absorption mode and operate in the regeneration mode if a humidity change amount of the second module chamber 213 recognized by the first sensor 361 and a humidity change amount of the third module chamber 214 recognized by the second sensor 362 is less than or equal to a reference value.

In one embodiment, when the drying module DM operates in the regeneration mode, the controller 10 may control the drying module DM to stop operating in the regeneration mode if the humidity change amount of the second module chamber 213 recognized by the first sensor 361 and the humidity change amount of the third module chamber 214 recognized by the second sensor 362 is less than or equal to the reference value.

The shoes care device 1 according to one embodiment of the present invention further includes a third sensor 363 capable of measuring the amount of moisture adsorbed to the dehumidifying material 331, and the controller 10 may control the drying module DM to operate the regeneration mode until the amount of moisture measured by the third sensor 363 is less than or equal to a set value.

Specifically, the controller 10 may control all the heating parts 320 to operate until the amount of moisture measured by the third sensor 363 is less than or equal to the set value.

In this case, as illustrated in FIG. 11, the third sensor 363 may include a moisture sensor installed adjacent to the dehumidifying material 331 to measure the amount of moisture adsorbed to the dehumidifying material 331, and the type and number thereof may vary as necessary.

In this way, when the moisture adsorbed on the dehumidifying material 331 is sensed to exceed the reference value, the shoes care device 1 according to one embodiment of the present invention first regenerates all dehumidifying materials 331 until the moisture is less than or equal to the reference value. Accordingly, the dehumidifying material 331 can maintain an appropriate state for dehumidification all the times even when the shoes care device 1 operates to refresh the shoes.

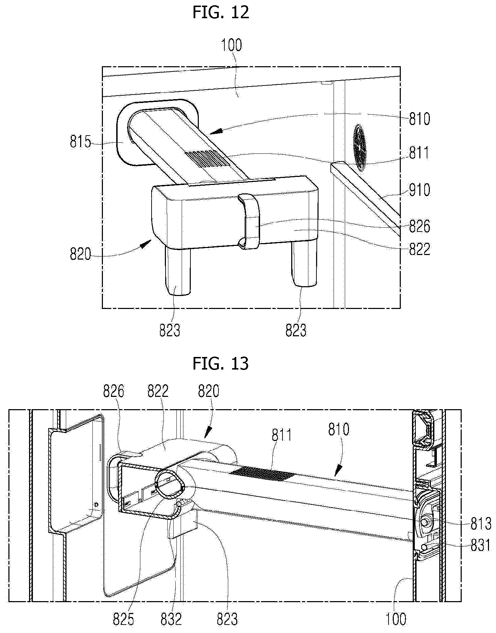

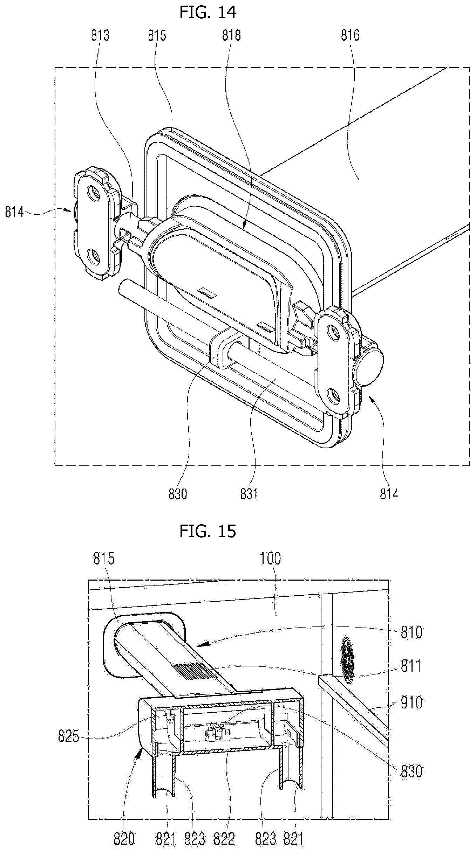

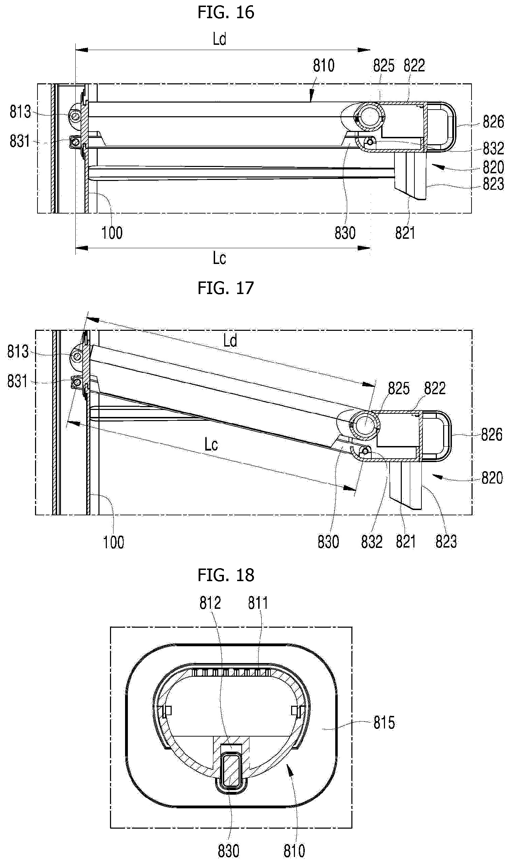

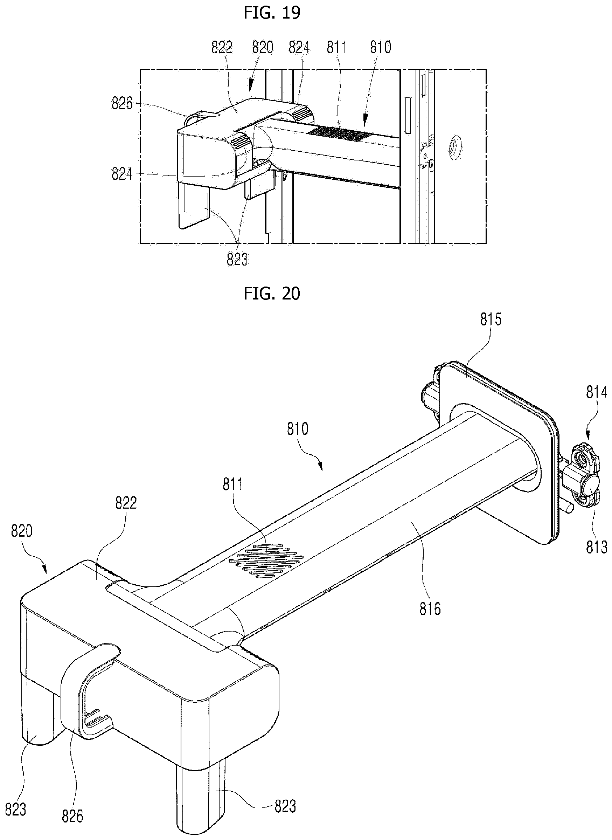

FIG. 12 is a view illustrating in more detail a nozzle duct 810 and a nozzle 820 installed in the inner cabinet 100 in the shoe care device 1 according to one embodiment of the present invention. FIG. 13 is a cross-sectional view illustrating coupling structures at one end and the other end of the nozzle duct 810 illustrated in FIG. 12. FIG. 14 is a cross-sectional view illustrating a portion where the nozzle duct 810 in FIG. 12 is coupled to the inner cabinet 100. FIG. 15 is a cross-sectional view illustrating a portion where the nozzle duct 810 of FIG. 12 is coupled to the nozzle 820. FIGS. 16 and 17 are views illustrating the state in which the angle of the nozzle duct 810 of FIG. 12 is adjusted. FIG. 18 is a cross-sectional view illustrating the interior of the nozzle duct 810 of FIG. 12. FIG. 19 is a view illustrating the nozzle duct 810 and the nozzle 820 of FIG. 12 viewed in another direction.

The shoe care device 1 according to one embodiment of the present invention may include an inner cabinet 100, an outlet 203 , a nozzle duct 810, a nozzle 820, a nozzle connector 830, a connection path F10, a blowing part 310, and a dehumidifying part 330.

The nozzle duct 810 is a part that is hinged to the inner cabinet 100 at one end thereof and protrudes into the inner cabinet 100 to form an air passage. The nozzle 820 is coupled to the other end of the nozzle duct so that the nozzle duct may form a passage through which air moves to the nozzle 820.

Since the nozzle duct 810 is installed to protrude into the inner cabinet 100, the position where the nozzle 820 is disposed inside the inner cabinet 100 may be varied.

In particular, the one end of the nozzle duct 810 may be hinged to the inner cabinet 100. That is, a nozzle supporter hinge axis 813 provided at one end of the nozzle duct 810 may be coupled to the inner cabinet 100 to make the nozzle duct 810 rotatable around the nozzle supporter hinge axis 813.

The nozzle 820 is a part that is hinged to the other end of the nozzle duct 810 to be insertable into a shoe from inside the inner cabinet 100 to spray air into the shoe. The air passing through the connection path F10 may be sprayed to the shoe inside the inner cabinet 100 through the nozzle 820.

The nozzle 820 sprays air in the state of being inserted into the shoe, thereby ensuring that air is smoothly introduced into the shoe.

In particular, the nozzle 820 may be hinged to the other end of the nozzle duct 810. That is, since the nozzle 820 is coupled to the nozzle hinge axis 825 formed at the other end of the nozzle duct 810, the nozzle 820 is rotatable around the nozzle hinge axis 825.

The nozzle connector 830 is a part that is installed separately from the nozzle duct 810, hinged to the inner cabinet 100 at one end, and hinged to the nozzle 820 at the other end. The nozzle connector may ensure that the angle of the nozzle 820 is maintained constant even when the nozzle duct 810 performs hinge rotation.

In relation to this, when the nozzle duct 810 performs hinge rotation around the nozzle supporter hinge axis 813 in a hinged manner, the position of the nozzle 820 coupled to the other end of the nozzle duct 810 is lowered.

In this case, when the angle of the nozzle 820 is also constrained by the angle of the nozzle duct 810, the nozzle 820 is also inclined integrally with the nozzle duct 810, making it difficult for the nozzle 820 to be inserted into the upper surface of a shoe.

Therefore, even when the nozzle duct 810 is disposed to be inclined, it may be desirable to adjust the angle of the nozzle 820 such that the nozzle is not inclined by a predetermined angle or more.

However, since the nozzle 820 is also hinged to the other end of the nozzle duct 810, a user may arbitrarily adjust the angle of the nozzle 820 such that the nozzle 820 is not inclined by a predetermined angle or more.

However, when the user performs operations as described above, there are problems in that it may be very inconvenient from the user's point of view and that the angle of the nozzle duct 810 may also be changed in the process of adjusting the angle of the nozzle 820.

Accordingly, with the nozzle connector 830 installed separately from the nozzle duct 810, even when the user does not separately adjust the angle of the nozzle 820, it may be desirable for the nozzle 820 to always maintain its angle constant in the process of adjusting only the angle of the nozzle duct 810.

Specifically, it may be desirable for the nozzle 820 to maintain the angle at which the lower discharge port 821 discharges air in the vertical direction.

In this case, the first connection hinge axis 831 formed at one end of the nozzle connector 830 is coupled to the inner cabinet 100, so that the nozzle connector 830 can also be rotated around the first connection hinge axis 831.

In addition, the nozzle 820 is coupled to the second connection hinge axis 832 formed at the other end of the nozzle connector 830, so that the nozzle 820 can also be rotated around the second connection hinge axis 832.

In addition, when the angle of the nozzle duct 810 is changed, the angle of the nozzle connector 830 may be changed correspondingly.

In this case, since the gap and lateral displacement between the nozzle supporter hinge axis 813 and the first connection hinge axis 831 are constrained to the inner cabinet 100, the nozzle supporter hinge axis 813 and the first connection hinge axis 831 may maintain the distance therebetween even when the nozzle duct 810 and the nozzle connector 830 rotate by the same angle.

Meanwhile, since the distance between the nozzle hinge axis 825 coupled to the nozzle 820 and the second connection hinge axis 832 is also constrained to the nozzle 820, even when the nozzle duct 810 and the nozzle connector 830 are rotated by the same, the nozzle hinge axis 825 and the second connection hinge axis 832 may also maintain the distance therebetween.

However, depending on the rotation of the nozzle duct 810, the lateral position of the nozzle hinge axis 825 may move toward the nozzle supporter hinge axis 813, and the longitudinal position of the nozzle hinge axis 825 may move downward.

At the same time, the lateral and longitudinal positions of the second connection hinge axis 832 may be moved to correspond to the lateral and longitudinal positions of the nozzle hinge axis 825.

Accordingly, since the nozzle hinge axis 825 and the second connection hinge axis 832 may move the lateral and longitudinal positions thereof equally while maintaining the same distance therebetween, even when the nozzle duct 810 and the nozzle connector 830 are changed to any angle, the angle of the nozzle 820 may be maintained constant.

In this way, in the shoe care device 1 according to the present embodiment, one end and the other end of the nozzle duct 810 are hinged to the inner cabinet 100 and the nozzle 820, respectively, and separately from the nozzle duct 810, one end and the other end of the nozzle connector 830 are hinged to the inner cabinet 100 and the nozzle 820, respectively, whereby even when the angle of the nozzle duct 810 is changed, the angle of the nozzle 820 is maintained constant. Therefore, shoe processing efficiency can be further improved by stably supplying air to a shoe.

In the shoe care device 1 according to one embodiment of the present invention, the nozzle 820 may include a nozzle body 822 hinged to the nozzle duct 810, and a nozzle protrusion 823 protruding downward from the nozzle body 822 and having a lower discharge port 821 provided at an end thereof.

Specifically, the nozzle body 822 is a part that is coupled to the nozzle duct 810 by the nozzle hinge axis 825 and is also coupled to the nozzle connector 830 by the second connection hinge axis 832.

Accordingly, even when the angle of the nozzle duct 810 is changed, the angle of the nozzle body 822 may be maintained constant.

Meanwhile, a nozzle handle 826 may be provided on the nozzle body 822. Thus, in order to adjust the angle of the nozzle duct 810, the user may easily adjust the angle of the nozzle duct 810 by holding the nozzle handle 826 and applying force without touching the nozzle duct 810.

The nozzle protrusion 823 is a part that protrudes from the nozzle body 822 to be easily inserted into a shoe, and may have a lower discharge port 821 that is open downward toward the shoe.

In this case, it may be desirable for the nozzle protrusion 823 to maintain a vertical angle such that the lower discharge port 821 discharges air in the vertical direction.

In addition, it is desirable to configure the end portion of the nozzle protrusion 823 to be inserted into an open surface of a shoe corresponding to an ankle of a shoe wearer and then to introduce air into the toe of the shoe smoothly.

To this end, the end portion of the nozzle protrusion 823 may be partially inclined.

As described above, in the shoe care device 1 according to the present embodiment, since the nozzle 820 includes the nozzle body portion 822 and the nozzle protrusion 823, the lower discharge port 821 provided in the nozzle protrusion 823 may be more easily inserted into the interior of the shoe.

In the shoe care device 1 according to one embodiment of the present invention, the length of the nozzle connector 830 may correspond to that of the nozzle duct 810.

As described above, since it is desirable for the nozzle 820 to maintain the angle at which the lower discharge port 821 discharges air in the vertical direction, the length Ld of the nozzle duct 810 and the length Lc of the nozzle connector 830 Lc are preferably equal to each other.

When the lengths of the nozzle duct 810 and the nozzle connector 830 are different, the lateral positions of the nozzle hinge axis 825 and the second connection hinge axis 832 are different. Therefore, it may be difficult for the nozzle 820, which is coupled to both the nozzle duct 810 and the nozzle connector 830, to maintain the angle at which air is discharged in the vertical direction.

In this way, in the shoe care device 1 according to the present embodiment, since the lengths of the nozzle connector 830 and the nozzle duct 810 correspond to each other, the nozzle 820, which is hinged to both the nozzle duct 810 and the nozzle connector 830, may always maintain the same angle.

In the shoe care device 1 according to one embodiment of the present invention, the nozzle duct 810 may have a nozzle groove 812 extending along the longitudinal direction, and the nozzle connector 830 may be installed by being inserted into the nozzle groove 812.

As described above, in order to make the angle of the nozzle duct 810 adjustable while the nozzle 820 maintains a constant angle, it is necessary to install the nozzle connector 830 separately from the nozzle duct 810.

However, although the nozzle connector 830 is a necessary member for the function of the shoe care device 1, it may be perceived as a secondary member from a user's point of view. In particular, when the nozzle connector 830 is exposed to the user, its appearance may not be good, and the structure of the shoe care device 1 may be perceived as complicated.

Accordingly, it is desirable for the nozzle connector 830 to perform the above-described functions without problems while being as little exposed to the user as possible.

Accordingly, the nozzle groove 812 may be provided in the nozzle duct 810 to correspond to the shape of the nozzle connector 830. In addition, the nozzle connector 830 may be coupled to the inner cabinet 100 and the nozzle 820 in the state of being inserted into the nozzle groove 812.

Through this, the nozzle connector 830 may perform the function of maintaining the nozzle 820 at a constant angle without problems while being minimally exposed to the user.

In this way, in the shoe care device 1 according to the present embodiment, since the nozzle connector 830 is installed by being inserted into the nozzle groove 812 provided along the longitudinal direction of the nozzle duct 810, exposure of the nozzle connector 830 may be minimized while the user changes the nozzle duct 810.

In the shoe care device 1 according to one embodiment of the present invention, the nozzle duct 810 may have an upper discharge port 811 that is open upward. For example, the upper discharge port 811 is provided in a portion of the upper surface of the nozzle duct 810 so that some of the air moving into the nozzle duct 810 may be discharged to the accommodation space 101 through the upper discharge port 811.

Accordingly, since the air is discharged within the inner cabinet 100 through the upper discharge port 811 provided in the nozzle duct 810 as well as the lower discharge port 821 provided in the nozzle 820, the air may be discharged to various portions within the inner cabinet 100 , so that the processing efficiency of a shoe can be further improved.

In the shoe care device 1 according to one embodiment of the present invention, an auxiliary discharge port 824 may be provided in the upper surface of the nozzle body 822. The auxiliary discharge port 824 is provided in a portion of the upper surface of the nozzle body 822, so that some of the air moving inside the nozzle duct 810 may be discharged into the accommodation space 101 through the auxiliary discharge port 824.

In this case, the auxiliary discharge port 824 may be provided in the upper surface of the nozzle body 822 toward the rear wall of the inner cabinet 100.

Inside the inner cabinet 100, moisture generated during a shoe processing process may be condensed on the inner wall or the like of the inner cabinet 100. In particular, when steam is supplied to the accommodation space 101, a relatively large amount of moisture may be condensed, and it is necessary to effectively remove the condensed moisture for the function of the shoe care device 1.

Meanwhile, a door 30 may be installed on the front surface of the inner cabinet 100, and, if necessary, an outlet 203 may be placed on the front side of the inner cabinet 100.

Accordingly, the front surface side in the interior of the inner cabinet 100 is relatively advantageous for air circulation, and the generated moisture may be effectively removed in the front surface side.

On the other hand, the rear wall side in the interior of the inner cabinet 100 is relatively disadvantageous for air circulation, and the generated moisture may not be effectively removed in the rear wall side.

Accordingly, by discharging air from the upper surface of the nozzle body 822 through the auxiliary discharge port 824 toward the rear wall of the inner cabinet 100, air may be circulated to the portion that is relatively vulnerable to air circulation.

In this way, since the shoe care device 1 according to the present embodiment also discharges air through the auxiliary discharge port 824 provided in the nozzle body 822 within the inner cabinet 100, dry air may be supplied to a portion of the interior of the inner cabinet 100 where moisture removable is disadvantageous.

In the shoe care device 1 according to one embodiment of the present invention, the nozzle body 822 may be attached to the ceiling surface of the inner cabinet 100 using magnetic force.

As described above, in the case where the nozzle duct 810 is installed to protrude to the interior of the inner cabinet 100, while the nozzle 820 is not in use, it may be desirable to position the nozzle 820 in the upper portion of the inner cabinet 100 so that the nozzle does not occupy the internal space of the inner cabinet 100.

In this case, the hinge axis resistance part 814 may maintain the nozzle 820 in the state of being positioned in the upper portion of the inner cabinet 100.

However, when an unintended external force is applied to the nozzle 820 or the performance of the hinge axis resistance part 814 deteriorates due to long-term use, there is a risk that the nozzle 820 may sag downward even when it is not in use.

Therefore, when the nozzle 820 is not used, the nozzle 820 may be attached to the ceiling surface of the inner cabinet 100 using magnetic force to hold and may be held on the ceiling surface of the inner cabinet 100.

In this case, a magnetic material may be disposed on at least one of the upper surface of the nozzle 820 and the ceiling surface of the inner cabinet 100, and the upper surface of the nozzle 820 and the ceiling of the inner cabinet 100 may be made of a material that can be attached by magnetic force.

In this way, in the shoe care device 1 according to the present embodiment, since the nozzle 820 is attached to the ceiling surface of the inner cabinet 100, the nozzle 820 and the nozzle duct 810, which are not in use, may be prevented from occupying the internal space of the inner cabinet 100 unnecessarily.

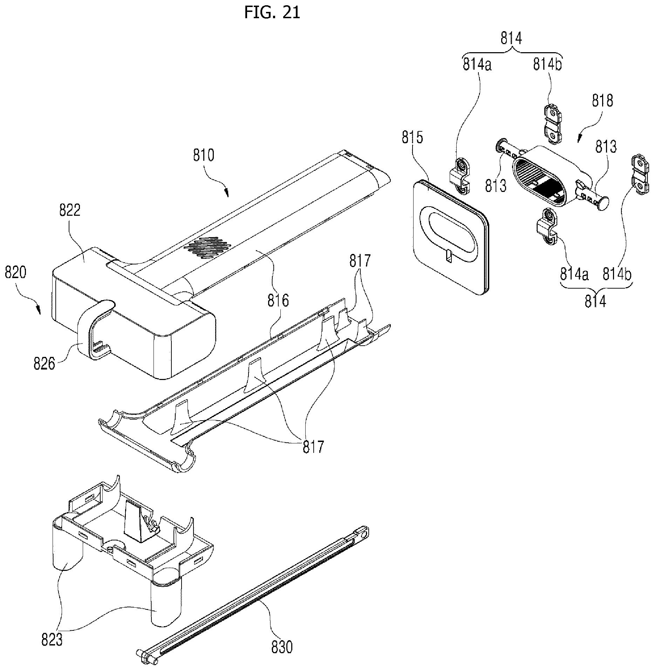

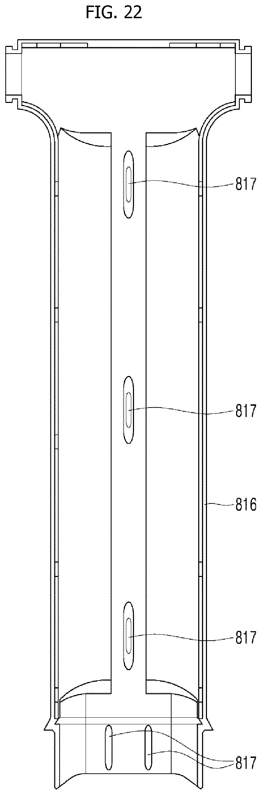

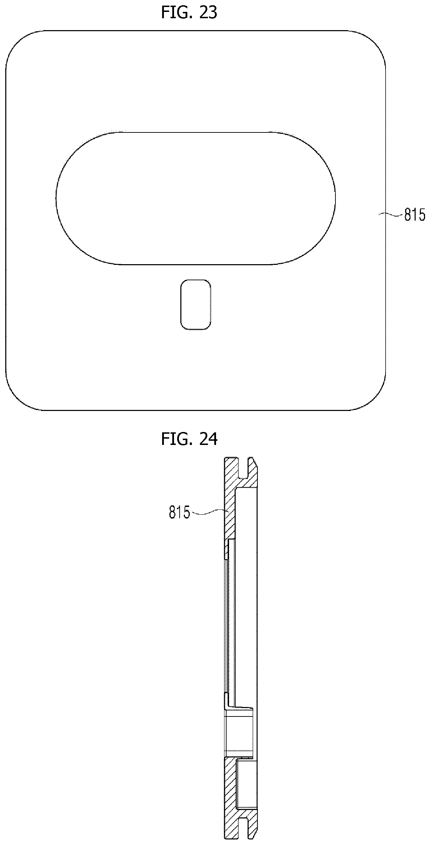

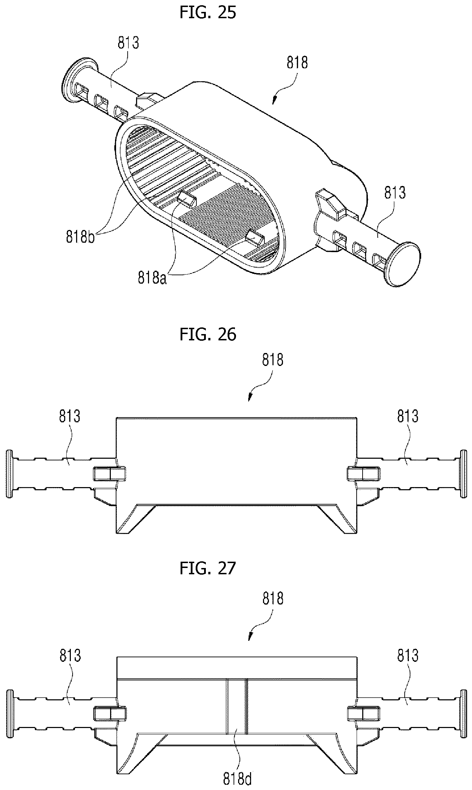

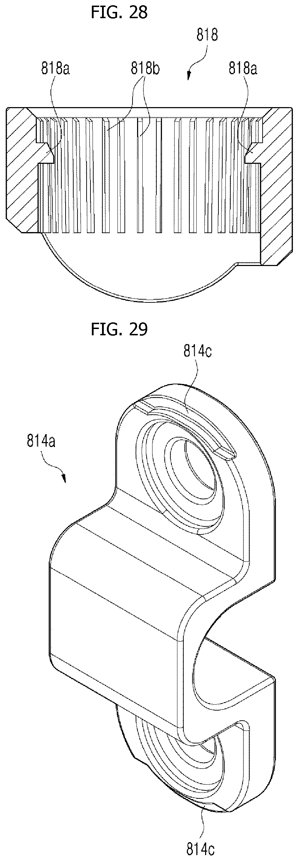

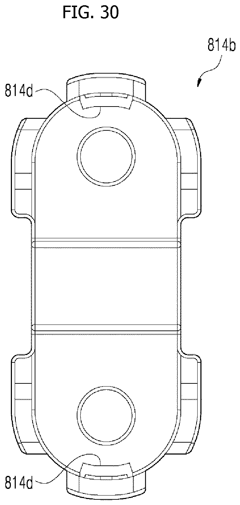

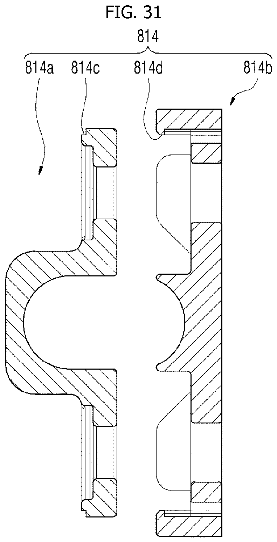

FIG. 20 is a view illustrating the nozzle duct 810 and the nozzle 820 of FIG. 12 viewed in another direction. FIG. 21 is an exploded view illustrating the nozzle duct 810 and the nozzle 820 of FIG. 20. FIG. 22 is a cross-sectional view illustrating the interior of the nozzle duct 810 of FIG. 20. FIGS. 23 and 24 are views illustrating the nozzle sealing part 815 of FIG. 20 in more detail. FIGS. 25 to 28 are views illustrating the nozzle supporter 818 of FIG. 20 in more detail. FIGS. 29 to 31 are views illustrating the hinge axis resistance part 814 of FIG. 20 in more detail.

The shoe care device 1 according to one embodiment of the present invention may include an inner cabinet 100, an outlet 203, a nozzle duct 810, a nozzle 820, a connection path F10, a blowing part 310, and a dehumidifying part 330. In this case, the nozzle duct 810 may include a nozzle bar 816 and a nozzle rib 817.

The inner cabinet 100 is a part that provides an accommodation space 101 configured to accommodate shoes, and have an outlet 203 and a nozzle 820 disposed therein.

The outlet 203 is a part provided in a portion of the inner cabinet 100 to allow the air inside the inner cabinet 100 to be sucked, and may form the beginning of the connection path F10.

In the outlet 203, a net, such as a grid or mesh, may be provided.

The outlet 203 may be configured in a shape extending to opposite side surfaces of the inner cabinet 100. That is, the outlet 203 may be provided in the form of a long hole extending from the bottom surface to the opposite side surfaces of the inner cabinet 100.

In this case, the bottom surface of the inner cabinet 100 may be inclined downward toward the outlet 203. That is, the portion where the outlet 203 is provided in the bottom surface of the inner cabinet 100 may be disposed at the lowest level. Accordingly, when there is water on the bottom surface of the inner cabinet 100, the water may flow along the bottom surface of the inner cabinet 100 due to gravity and flow into the outlet 203.

The nozzle duct 810 is a part that is hinged to the inner cabinet 100 at one end thereof and protrudes into the inner cabinet 100 to form an air passage. The nozzle 820 is coupled to the other end of the nozzle duct so that the nozzle duct may form a passage through which air moves to the nozzle 820.

The nozzle 820 is a part that is hinged to the other end of the nozzle duct 810 to be insertable into a shoe from inside the inner cabinet 100 to spray air into the shoe. The air passing through the connection path F10 may be sprayed to the shoe inside the inner cabinet 100 through the nozzle 820.

The connection path F10 is a part that forms a flow path through which air in the accommodation space 101 is discharged from the inner cabinet 100 and then flows back to the accommodation space 101. The connection path may be an air flow path through which the air inside the inner cabinet 100 is dehumidified while being suctioned into the module chamber 210 and then blown to pass through the dehumidifying part 330, and is supplied back into the inner cabinet 100.

In this case, the connection path F10 is a part where air circulates between the outlet 203 and the nozzle 820. The outlet 203 may form the inlet of the connection path F10, and the nozzle 820 may form the outlet of the connection path F10.

The blowing part 310 is a part disposed in the connection path F10 to blow air. When the blowing part 310 is operated, air may be suctioned from the inner cabinet 100, and the suctioned air may be blown from the connection path F10.

In this case, the blowing part 310 may be installed on the connection path F10 and blow air from the outlet 203 toward the nozzle 820.

The dehumidifying part 330 is a part that is installed on the connection path F10 and dehumidifies air. With the heating of the dehumidifying material 430, the moisture adsorbed on the dehumidifying material 430 may be separated and the dehumidifying material 430 may be regenerated to the state in which the dehumidifying function can be performed.

The module chamber 210 is a part that blows air from the accommodation space 101 and is capable of heating the dehumidifying part 330, which is disposed on the path of the blown air.

In this case, the dehumidifying part 330 is heated, whereby the moisture adsorbed on the dehumidifying part 330 is separated, so that the dehumidifying part 330 can be regenerated to the state in which the dehumidifying function can be performed.

To this end, the module chamber 210 may include a dehumidifying part 330, a blowing part 310, a heating part 320, a damper 350, and the like.

In particular, the module chamber 210 may form a portion of the connection path F10 through which air circulates between the outlet 203 and the nozzle 820, and the regeneration path F20, which is branched from the connection path F10 passing through the dehumidifying part 330, so that blowing is performed therethrough.

Accordingly, the air in the accommodation space 101 may move to the connection path F10 or the regeneration path F20 in the process of being blown to the module chamber 210 to pass through the dehumidifying part 330.

The controller 10 is a part that controls the shoe care device 1, and may control the module chamber 210 to selectively open/close the connection path F10 and the regeneration path F20 depending on whether the dehumidifying part 330 is heated.

That is, the controller 10 may selectively open/close the connection path F10 and the regeneration path F20 depending on whether the dehumidifying part 330 is in a regenerated state.

The connection path F10 is a part where air circulates between the outlet 203 and the nozzle 820. The outlet 203 may form the inlet of the connection path F10, and the nozzle 820 may form the outlet of the connection path F10.

That is, the connection path F10 may be an air flow path through which the air inside the inner cabinet 100 is dehumidified in the process of being sucked into the module chamber 210 and then blown to pass through the dehumidifying part 330, and is then returned again to the interior of the inner cabinet 100.

In this way, the shoe care device 1 according to the present embodiment may always maintain appropriate shoe processing performance since the dehumidifying part 330 is disposed in the module chamber 210 not only to collect moisture and bacteria in the blown air, but also to heat and regenerate the dehumidifying part 330 in the module chamber 210.

In addition, the shoe care device 1 according to the present embodiment may prevent a user from being exposed to the air used to dehumidify and deodorize shoes since the connection path F10 through which air circulates is provided between the outlet 203 and the nozzle 820 each of which is disposed inside the inner cabinet 100.

The nozzle bar 816 is a part that is provided in a tubular shape extending along the longitudinal direction through which air is movable along the internal space. The nozzle bar may connect the inner cabinet 100 and the nozzle 820 to provide an air moving passage.

In this case, since the nozzle bar 816 has a hollow tubular structure, sufficient rigidity against an external force or the like may not be ensured. When the rigidity of the nozzle bar 816 is insufficient, the nozzle duct 810 may be deformed or damaged when manufacturing the shoe care device 1.

In addition, when the nozzle bar 816 is not sufficiently rigid, the function of the nozzle duct 810 may not be performed smoothly in the process of using the shoe care device 1, and problems in usability may occur.

Therefore, in order to appropriately reinforce the rigidity of the nozzle bar 816, it may be desirable to form the nozzle rib 817 inside the nozzle bar 816.

The nozzle rib 817 is a portion provided inside the nozzle bar 816 to connect the upper and lower surfaces to each other, and may reinforce the rigidity of the nozzle bar 816 to minimize deformation and damage even against an external force.

Nozzle ribs 817 may be disposed at a plurality of points with a predetermined length along the longitudinal direction of the nozzle bar 816 so as not to impede the movement of air inside the nozzle bar 816.

In addition, the nozzle ribs 817 may be arranged in parallel at portions where the nozzle supporter 818 and the nozzle bar 816 are coupled to further reinforce rigidity.

In this way, since the shoe care device 1 according to the present embodiment are provided with the nozzle ribs 817 that connect the upper and lower surfaces inside the tubular nozzle bar 816 to reinforce rigidity, the nozzle duct 810 may have sufficient rigidity to prevent functional deterioration or inconvenience in use in the process of manufacturing and using the shoe care device 1.

The shoe care device 1 according to one embodiment of the present invention may further include a steam generator 700 configured to supply steam to the inner cabinet 100.

That is, since steam treatment of shoes is performed by supplying steam into the inner cabinet 100 through the steam generator 700, a refreshing effect through swelling of the shoe material or the like may be achieved in addition to a sterilization effect by the high temperature of steam.

In the shoe care device 1 according to one embodiment of the present invention, the nozzle duct 810 may be installed to protrude forward from the rear wall of the inner cabinet 100.

That is, since the nozzle duct 810 is installed to be directed forward from the rear wall of the inner cabinet 100, the internal space of the inner cabinet 100 may be effectively utilized.

In the shoe care device 1 according to one embodiment of the present invention, one end of the nozzle duct 810 may be coupled while penetrating the inner cabinet 100.

As described above, the air in the accommodation space 101 may move to the connection path F10 or the regeneration path F20 in the process of being discharged to the exterior of the inner cabinet 100 through the outlet and then blown into the module chamber 210 to pass through the dehumidifying part 330.

During this, the air moving to the connection path F10 may be discharged back into the accommodation space 101 through the nozzle duct 810 and the nozzle 820 installed inside the inner cabinet 100.

Therefore, in order for air to circulate along the connection path F10, a portion connected to the nozzle duct 810 from the exterior of the inner cabinet 100 to allow air to move therethrough is required.

Meanwhile, since one end of the nozzle duct 810 is hinged to the inner cabinet 100 as described above, it may be desirable for air to move from the exterior of the inner cabinet 100 to the nozzle duct 810 through the portion coupled to the inner cabinet 100 as described above.

Accordingly, one end of the nozzle duct 810 hinged to the inner cabinet 100 may be coupled while penetrating the inner cabinet 100, allowing air outside the inner cabinet 100 to flow into the nozzle duct 810.

In this way, in the shoe care device 1 according to the present embodiment, since the nozzle duct 810 is coupled to the inner cabinet 100 in a penetrating structure, air may move smoothly from the connection path F10 to the nozzle duct 810.

In the shoe care device 1 according to one embodiment of the present invention, the nozzle duct 810 may include a nozzle sealing part 815 interposed to surround the outer peripheral surface of the nozzle duct 810 in the portion coupled with the inner cabinet 100.

As described above, when the nozzle duct 810 is coupled by causing one end thereof to penetrate the inner cabinet 100, an airtightness problem may occur at this coupled portion.

In particular, since the nozzle duct 810 is coupled to the inner cabinet 100 to be capable of hinge rotation about one end, a structure that is capable of maintaining airtightness without interfering with the hinge rotation of the nozzle duct 810 is required.

Accordingly, the nozzle sealing part 815 may be coupled to surround the outer peripheral surface of the nozzle duct 810 to prevent air from moving to the outer peripheral surface of the nozzle duct 810.

In addition, the nozzle sealing part 815 may be made of a certain elastic material member to minimize interference with the hinge rotation of the nozzle duct 810.

In this way, in the shoe care device 1 according to the present embodiment, since the nozzle duct 810 is sealed in the coupling surface with the inner cabinet 100 other than the penetration portion by the nozzle sealing part 815, it is possible to prevent air from leaking to parts other than the connection path F10 while ensuring a certain degree of mobility of the nozzle duct 810 with respect to the inner cabinet 100.

Meanwhile, during the hinge rotation of the nozzle duct 810, the nozzle sealing part 815 may press the nozzle duct 810 using an elastic restoring force. Accordingly, a force to return the nozzle duct 810 to a horizontal state may be applied to the nozzle duct 810 by the nozzle sealing part 815.

However, as described above, when a frictional force generated by the hinge axis resistance part 814 is greater than the elastic restoring force of the nozzle sealing part 815, the nozzle duct 810 may maintain its current angle state without returning to the horizontal state.

In the shoe care device 1 according to one embodiment of the present invention, the portion of the nozzle sealing part 815 that is in contact with the outer peripheral surface of the nozzle bar 816 may be formed relatively thinner than the remaining portion.

As described above, it is necessary for the nozzle sealing part 815 to have a structure that is capable of maintaining airtightness without interfering with the hinge rotation of the nozzle duct 810.

In this case, the nozzle sealing part 815 may be made of an elastic material member to allow the hinge rotation of the nozzle duct 810 to a certain extent.

However, in order to allow the hinge rotation of the nozzle duct 810 to be performed more smoothly, it may be desirable to make the thickness of the nozzle sealing part 815 at the portion in contact with the outer peripheral surface of the nozzle bar 816 as thin as possible within the limit of maintaining airtightness.

In this way, in the shoe care device 1 according to the present embodiment, since the nozzle sealing part 815 in contact with the outer peripheral surface of the nozzle bar 816 is made relatively thin, interference with the nozzle sealing unit 815 may be minimized when adjusting the angle of the nozzle duct 810 while airtightness is maintained.

In the shoe care device 1 according to one embodiment of the present invention, the nozzle duct 810 may further include a nozzle supporter 300, which is fastened to the end portion of the nozzle bar 816 that penetrates the nozzle sealing part 815, to support the nozzle bar 816 with respect to the inner cabinet 100.

That is, the end portion of the nozzle bar 816 may be coupled to the nozzle supporter 818, and the nozzle supporter 818 may be coupled to the inner cabinet 100 to support the nozzle bar 816.

As described above, since the nozzle bar 816 is made of a hollow tubular member, it may not be desirable in terms of rigidity for the end portion of the nozzle bar 816 to be directly coupled to the inner cabinet 100.

Therefore, when the nozzle bar 816 is indirectly coupled to the inner cabinet 100 via the nozzle supporter 818 having a predetermined rigidity, the nozzle duct 810 may be more stably and firmly coupled to the inner cabinet 100.

In this way, in the shoe care device 1 according to the present embodiment, since the nozzle duct 810 is supported with respect to the inner cabinet 100 via the nozzle supporter 818, the nozzle duct 810 may be coupled to the inner cabinet 100 more stably.

In the shoe care device 1 according to one embodiment of the present invention, the nozzle supporter 818 may include a nozzle supporter hooks 818a that protrude inward from the upper and lower surfaces of the portion fastened to the nozzle bar 816, respectively.

When the end portion of the nozzle bar 816, which is a tubular member extending along the longitudinal direction, is simply fitted into the nozzle supporter 818, there is a risk that the nozzle bar 816 may be separated from the nozzle supporter 818 by an external force in the longitudinal direction.

Therefore, it may be desirable to provide the nozzle supporter hooks 818a at the coupling portion between the nozzle bar 816 and the nozzle supporter 818 to resist the external force in the longitudinal direction.

Accordingly, when the outer peripheral surface of the nozzle bar 816 is fitted into the inner peripheral surface of the nozzle supporter 818, the nozzle supporter hooks 818a provided on the nozzle supporter 818 may be fitted and fastened to the outer peripheral surface of the nozzle bar 816.

In this way, in the shoe care device 1 according to the present embodiment, since the nozzle supporter hooks 818a provided on the nozzle supporter 818 are fastened to the outer peripheral surface of the nozzle bar 816, the coupling between the nozzle bar 816 and the nozzle supporter 818 may be maintained more stably.

In the shoe care device 1 according to one embodiment of the present invention, the nozzle supporter 818 may further include a nozzle supporter rib 818b provided along the longitudinal direction of the nozzle bar 816 on the inner surface of the portion fastened to the nozzle bar 816.

As described above, since it is necessary for the nozzle duct 810 to perform hinge rotation in the state in which the nozzle bar 816 is coupled to the nozzle supporter 818, relatively large stress and deformation may occur in the portion where the nozzle bar 816 and the nozzle supporter 818 are coupled.

Therefore, it may be desirable to structurally reinforce the portion where the nozzle bar 816 and the nozzle supporter 818 are coupled to appropriately respond to applied stress and deformation.

Accordingly, by providing the nozzle supporter rib 818b in a concavo-convex structure on the inner surface of the nozzle supporter 818 coupled to the nozzle bar 816, greater structural rigidity may be achieved.

In this way, in the shoe care device 1 according to the present embodiment, since the nozzle supporter rib 818b is provided on the inner surface of the nozzle supporter 818 along the longitudinal direction of the nozzle bar 816, the rigidity of the nozzle supporter 818 coupled to the nozzle bar 816 may be reinforced.

In the shoe care device 1 according to one embodiment of the present invention, the nozzle supporter 818 may include a nozzle supporter hinge axis 813 and a hinge axis resistance part 814.

Specifically, the nozzle supporter hinge axis 813 is a part installed to enable hinge rotation at a portion coupled with the inner cabinet 100. The nozzle supporter hinge axis 813 provided in the nozzle supporter 818 is coupled to the inner cabinet 100 to make the nozzle duct 810 rotatable about the nozzle supporter hinge axis 813.

In this case, the angle of the nozzle duct 810 may be easily changed via the nozzle supporter hinge axis 813, but in order to maintain the nozzle duct 810 at the angle desired by the user, a configuration that limits the rotation of the nozzle supporter hinge axis 813 may be required.

When this configuration is not present, the nozzle duct 810 may not be maintained at the angle desired by the user, which may cause the nozzle 820 to sag downward due to gravity.

In addition, it may be impossible to hold the nozzle 820 at the uppermost portion within the accommodation space 101 when the nozzle 820 is not in use.

In particular, the nozzle duct 810 is has a structure in which one end is hinged to the inner cabinet 100, but the other end does not have a separate support member, in which since sagging due to the own weight of the nozzle duct inevitably increases toward the other end, it may be said that a separate configuration is required to prevent such sagging.

The hinge axis resistance part 814 is a part installed to limit the rotation of the nozzle supporter hinge axis 813, and may restrict the nozzle supporter hinge axis 813 from rotating at a predetermined pressure or lower.

Therefore, only when the user applies an external force of the predetermined pressure or lower, the nozzle supporter hinge axis 813 may rotate so that the angle of the nozzle duct 810 may be changed. In addition, when an external force of the predetermined pressure or lower is not applied, the rotation is limited by the hinge axis resistance part 814 so that the angle of the nozzle duct 810 may be maintained.

As described above, in the shoe care device 1 according to the present embodiment, the nozzle supporter 818 is hinged to the inner cabinet 100 via the nozzle supporter hinge axis 813, and the nozzle supporter 818 is supported on the inner cabinet 100 via the hinge axis resistance part 814. Thus, if necessary, the angle of the nozzle duct 810 may be adjusted through hinge rotation relative to the inner cabinet 100, and the angle of the nozzle duct 810 may be maintained constant when the angle change of the nozzle duct 810 is not intended.

In the shoe care device 1 according to one embodiment of the present invention, the hinge axis resistance part 814 may cover the nozzle supporter hinge axis 813 and may be coupled to the inner cabinet 100 to generate a frictional force on the nozzle supporter hinge axis 813.

That is, the hinge axis resistance part 814 may be coupled to the inner cabinet 100, and the nozzle supporter hinge axis 813 may be interposed between the coupling surfaces of the hinge axis resistance part 814 and the inner cabinet 100.

Therefore, the frictional force may be generated between the nozzle supporter hinge axis 813 and the hinge axis resistance part 814 due to the close contact force with which the hinge axis resistance part 814 is coupled to the inner cabinet 100.

As a result, in the case where a pressure smaller than the frictional force generated by the hinge axis resistance part 814 is applied when the nozzle supporter hinge axis 813 is about to rotate, the rotation of the nozzle supporter hinge axis 813 may be limited.

In this case, the hinge axis resistance part 814 may be made of a member containing an elastic material, and may be made of a material with excellent strength and elasticity, such as acetal (POM).

In this way, in the shoe care device 1 according to the present embodiment, since the hinge axis resistance part 814 generates a frictional force on the nozzle supporter hinge axis 813, when an external force smaller than the generated frictional force is applied, the angle of the nozzle duct 810 may be maintained to achieve a stable state.

In the shoe care device 1 according to one embodiment of the present invention, a pair of nozzle supporter hinge axes 813 may be installed symmetrically on opposite side surfaces of the nozzle supporter 818.

As described above, the nozzle duct 810 has a structure in which one end is hinged to the inner cabinet 100, but the other end does not have a separate support member, in which sagging due to the own weight of the nozzle duct inevitably increases toward the other end.

In particular, when a torsional deformation occurs toward the other end of the nozzle duct 810 in addition to the sagging, there is a risk that the performance of the shoe care device 1 may deteriorate.

Therefore, in order to prevent torsional deformation, it is necessary to arrange the nozzle supporter hinge axes 813 symmetrically to prevent eccentricity from occurring toward the opposite side surfaces of the nozzle duct 810.

In this way, in the shoe care device 1 according to the present embodiment, since a pair of nozzle supporter hinge axes 813 are installed on the opposite side surfaces of the nozzle supporter 818, eccentricity that occurs when the hinge of the nozzle duct 810 rotates may be minimized.

In the shoe care device 1 according to one embodiment of the present invention, the hinge axis resistance part 814 may be installed on each nozzle supporter hinge axis 813. That is, the hinge axis resistance part 814 may be installed on each nozzle supporter hinge axis 813 to generate a frictional force.

When a pair of nozzle supporter hinge axes 813 are installed on the opposite side surfaces of the nozzle supporter 818, the frictional force that limits the rotation of the nozzle supporter hinge axes 813 also needs to be generated uniformly on the opposite side surfaces.

Otherwise, there is a risk that eccentricity may occur as described above and cause torsional deformation in the nozzle duct 810.

In this way, in the shoe care device 1 according to the present embodiment, since the hinge axis resistance part 814 is installed on each nozzle supporter hinge axis 813, a greater support force for the nozzle duct 810 may be ensured.

In the shoe care device 1 according to one embodiment of the present invention, the hinge axis resistance part 814 may include a first hinge axis holder 814a and a second hinge axis holder 814b.

Specifically, the first hinge axis holder 814a is a part that covers one surface of the nozzle supporter hinge axis 813, and may be in close contact with a portion of the outer peripheral surface of the nozzle supporter hinge axis 813.

The second hinge axis holder 814b is a part that covers the other surface of the nozzle supporter hinge axis 813 and is coupled to the first hinge axis holder 814a, and may be in close contact with the remaining portion of the outer peripheral surface of the nozzle supporter hinge axis 813.

That is, the hinge axis resistance part 814 may be coupled to the nozzle supporter hinge axis 813 by coupling the first hinge axis holder 814a and the second hinge axis holder 814b to each other in the state in which the nozzle supporter hinge axis 813 is interposed therebetween.

In this case, the first hinge axis holder 814a and the second hinge axis holder 814b may be coupled using separate fastening members or the like, and the first hinge axis holder 814a and the second hinge axis holder 814b may be formed in a shape corresponding to the axial cross-sectional shape of the nozzle supporter hinge axis 813.

In this way, in the shoe care device 1 according to the present embodiment, since the hinge axis resistance part 814 includes the first hinge axis holder 814a and the second hinge axis holder 814b, the nozzle supporter hinge axis 813 and the hinge axis resistance part 814 may be coupled more easily.

In the shoe care device 1 according to one embodiment of the present invention, a hinge axis holder groove 814c may be provided on the outer surface of the first hinge axis holder 814a, and a hinge axis holder hook 814d, which is fastenable to the hinge axis holder groove 814c, may be provided on the outer surface of the second hinge axis holder 814b.

As described above, when the first hinge axis holder 814a and the second hinge axis holder 814b are coupled using separate fastening members or the like, the number of fastening members is inevitably limited, and there is a risk that a gap may occur between the first hinge axis holder 814a and the second hinge axis holder 814b in the portion where the fastening members are not disposed.

When a gap occurs between the first hinge axis holder 814a and the second hinge axis holder 814b as described above, the frictional force between the hinge axis resistance part 814 and the nozzle supporter hinge axis 813 may decrease, making it difficult for the function of the hinge axis resistance part 814 to be performed properly.

Accordingly, it may be desirable to further improve the fastening force by providing hook and groove structures, which are fastenable to each other, on the outer surfaces of the first hinge axis holder 814a and the second hinge axis holder 814b.

In this way, in the shoe care device 1 according to the present embodiment, since the hinge axis holder hook 814d provided on the second hinge axis holder 814b is fastened to the hinge axis holder groove 814c provided on the first hinge axis holder 814a, the coupling between the first hinge axis holder 814a and the second hinge axis holder 814b may be maintained more stably.

The shoe care device 1 according to one embodiment of the present invention may include a nozzle connector 830 that is installed separately from the nozzle duct 810, hinged to the inner cabinet 100 at one end, and hinged to the nozzle 820 at the other end.

That is, one end and the other end of the nozzle duct 810 are hinged to the inner cabinet 100 and the nozzle 820, respectively, and, separately from the nozzle duct 810, one end and the other end of the nozzle connector 830 are hinged to the inner cabinet 100 and the nozzle 820, respectively. Thus, the angle of the nozzle 820 may be maintained constant even when the angle of the nozzle duct 810 is changed.

In the shoe care device 1 according to one embodiment of the present invention, the nozzle connector 830 may be disposed under the nozzle supporter 818, and the nozzle supporter 818 may have a nozzle supporter groove 818d provided on the lower surface thereof to correspond to the shape of the upper surface shape of the nozzle connector 830.

As described above, it is desirable for the nozzle connector 830 to perform the above-described functions without problems while being as little exposed to the user as possible.

Accordingly, the nozzle connector 830 may be disposed under the nozzle bar 816, and accordingly, the nozzle connector 830 may be disposed under the nozzle supporter 818.

In this case, depending on the hinge rotation of the nozzle duct 810, the nozzle connector 830 and the nozzle supporter 818 also move to a predetermined angle, and in that process, there is a risk that interference between the nozzle supporter 818 and the nozzle connector 830 may occur.

Therefore, it may be desirable to form the nozzle supporter groove 818d in the lower surface of the nozzle supporter 818 that is to come into contact with the upper surface of the nozzle connector 830 to correspond to the shape of the nozzle connector 830, and to ensure that the upper surface of the nozzle connector 830 is engaged with the nozzle supporter groove 818d.

In this way, in the shoe care device 1 according to the present embodiment, since the upper surface of the nozzle connector 830 is engaged with the nozzle supporter groove 818d provided in the lower surface of the nozzle supporter 818, interference between the nozzle connector 830 and the nozzle supporter 818 may be minimized.

Hereinabove, a specific embodiment of the present invention is described and illustrated, but the present invention is not limited to the disclosed embodiment, and it may be appreciated by those skilled in the art that the exemplary embodiment can be variously modified and transformed to another specific embodiment without departing from the spirit and the scope of the present invention. Therefore, the scope of the present invention will not be defined by the described embodiment, but defined by the technical spirit disclosed in the claims.

### INDUSTRIAL APPLICABILITY

By at least one of exemplary embodiments of the present invention, a dehumidifying part is disposed in a module chamber not only to collect moisture and bacteria in a ventilation air but also to regenerate the dehumidifying part by heating the dehumidifying part in the module chamber, thereby maintaining proper shoes treatment performance all the times.

In addition, by at least one of exemplary embodiments of the present invention, a connection path through which air circulates is formed between a outlet and a nozzle respectively disposed inside an inner cabinet, thereby preventing the air used for dehumidifying and deodorizing shoes from being exposed to the user.

In addition, by at least one of exemplary embodiments of the present invention, since a nozzle rib is provided to connect the upper and lower surfaces inside the tubular nozzle bar to reinforce rigidity, the nozzle duct may have sufficient rigidity to prevent functional deterioration or inconvenience in use in the process of manufacturing and using the shoe care device.

In addition, by at least one of exemplary embodiments of the present invention, since steam treatment of shoes may be performed by supplying steam into the inner cabinet, a refreshing effect through swelling of the shoe material or the like may be achieved in addition to a sterilization effect by the high temperature of steam.

In addition, by at least one of exemplary embodiments of the present invention, since the nozzle duct is installed from the rear wall of the inner cabinet toward the front, the internal space of the inner cabinet may be effectively utilized.

In addition, by at least one of exemplary embodiments of the present invention, since the nozzle duct is coupled to the inner cabinet in a penetrating structure, air may move smoothly from the connection path to the nozzle duct.

In addition, by at least one of exemplary embodiments of the present invention, since the nozzle duct is sealed on the coupling surface with the inner cabinet other than the penetration portion by the nozzle sealing part, air is prevented from leaking to parts other than the connection path while ensuring a certain degree of mobility of the nozzle duct with respect to the inner cabinet.

In addition, by at least one of exemplary embodiments of the present invention, since the nozzle sealing part in contact with the outer peripheral surface of the nozzle bar is made relatively thin, interference with the nozzle sealing part may be minimized when adjusting the angle of the nozzle duct while airtightness is maintained.

In addition, by at least one of exemplary embodiments of the present invention, since the nozzle duct is supported with respect to the inner cabinet via the nozzle supporter, the nozzle duct may be coupled to the inner cabinet more stably.

In addition, by at least one of exemplary embodiments of the present invention, since the nozzle supporter hooks provided on the nozzle supporter are fastened to the outer peripheral surface of the nozzle bar, the coupling between the nozzle bar and the nozzle supporter may be maintained more stably.

In addition, by at least one of exemplary embodiments of the present invention, since the nozzle supporter rib is provided on the inner surface of the nozzle supporter along the longitudinal direction of the nozzle bar, the rigidity of the nozzle supporter coupled to the nozzle bar may be reinforced.

In addition, by at least one of exemplary embodiments of the present invention, the nozzle supporter is hinged to the inner cabinet via the nozzle supporter hinge axis, and the nozzle supporter is supported on the inner cabinet via the hinge axis resistance part. Thus, if necessary, the angle of the nozzle duct may be adjusted through hinge rotation with respect to the inner cabinet, and the angle of the nozzle duct may be maintained constant when the angle change of the nozzle duct is not intended.

In addition, by at least one of exemplary embodiments of the present invention, the hinge axis resistance part generates a frictional force on the nozzle supporter hinge axis. Thus, when an external force smaller than the generated frictional force is applied, the angle of the nozzle duct may be maintained to achieve a stable state.

In addition, by at least one of exemplary embodiments of the present invention, since a pair of nozzle supporter hinge axes are installed on the opposite side surfaces of the nozzle supporter, eccentricity that occurs when the hinge of the nozzle duct rotates may be minimized.

In addition, by at least one of exemplary embodiments of the present invention, since the hinge axis resistance part includes the first hinge axis holder and the second hinge axis holder, the nozzle supporter hinge axis and the hinge axis resistance part may be coupled more easily.

In addition, by at least one of exemplary embodiments of the present invention, since the hinge axis holder hook provided on the second hinge axis holder is fastened to the hinge axis holder groove provided on the first hinge axis holder, the coupling between the first hinge axis holder and the second hinge axis holder may be maintained more stably.

In addition, according to at least one of exemplary embodiments of the present invention, since one end and the other end of the nozzle duct are hinged to the inner cabinet and the nozzle, respectively, and, separately from the nozzle duct, one end and the other end of the nozzle connector are hinged to the inner cabinet and the nozzle, respectively, the angle of the nozzle may be maintained constant even when the angle of the nozzle duct is changed.

In addition, according to at least one of exemplary embodiments of the present invention, since the upper surface of the nozzle connector is engaged with the nozzle supporter groove provided in the lower surface of the nozzle supporter, interference between the nozzle connector and the nozzle supporter may be minimized.

## Claims

1. A shoe care device comprising:
an inner cabinet having an accommodation space configured to accommodate a shoe;
an outlet through which air inside the inner cabinet is suctioned;
a nozzle duct having one end hinged to the inner cabinet so that the nozzle is installed to protrude into the inner cabinet and provide an air passage;
a nozzle coupled to another end of the nozzle duct to be inserted into the shoe within the inner cabinet to spray air into the shoe;
a connection path connected to the nozzle from the outlet;
a blowing part installed on the connection path to blow air from the outlet toward the nozzle; and
a dehumidifying part installed on the connection path to dehumidify the blown air,
wherein the nozzle duct comprises:
a nozzle bar provided in a tubular shape extending along a longitudinal direction and having an internal space through which air is movable; and
a nozzle rib provided to connect upper and lower surfaces inside the nozzle bar to each other.

2. The shoe care device of claim 1, further comprising:
a steam generator configured to supply steam to the inner cabinet.

3. The shoe care device of claim 1, wherein the nozzle duct is installed to protrude forward from a rear wall of the inner cabinet.

4. The shoe care device of claim 3, wherein one end of the nozzle duct penetrates the inner cabinet and is coupled thereto.

5. The shoe care device of claim 4, wherein the nozzle duct further comprises a nozzle sealing part interposed to surround an outer peripheral surface of the nozzle bar at a portion coupled to the inner cabinet.

6. The shoe care device of claim 5, wherein the nozzle sealing part is formed to be relatively thinner in a portion that is to come into contact with the outer peripheral surface than a remaining portion.

7. The shoe care device of claim 5, wherein the nozzle duct further comprises a nozzle supporter that is fastened to an end portion of the nozzle bar that penetrates the nozzle sealing part, the nozzle supporter being configured to support the nozzle bar with respect to the inner cabinet.

8. The shoe care device of claim 7, wherein the nozzle supporter comprises a nozzle supporter hook which protrudes inward from each of upper and lower surfaces of the portion fastened to the nozzle bar.

9. The shoe care device of claim 8, wherein the nozzle supporter further comprises a nozzle supporter rib provided along the longitudinal direction of the nozzle bar on an inner surface of a portion fastened to the nozzle bar.

10. The shoe care device of claim 7, wherein the nozzle supporter comprises:
a nozzle supporter hinge axis installed to enable hinge rotation at a portion coupled with the inner cabinet; and
a hinge axis resistance part installed to limit the rotation of the nozzle supporter hinge axis.

11. The shoe care device of claim 10, wherein the hinge axis resistance part covers the nozzle supporter hinge axis and is coupled to the inner cabinet to generate a frictional force on the nozzle supporter hinge axis.

12. The shoe care device of claim 11, wherein a pair of nozzle supporter hinge axes are installed symmetrically on opposite side surfaces of the nozzle supporter.

13. The shoe care device of claim 11, wherein the hinge axis resistance part comprises:
a first hinge axis holder that covers one surface of the nozzle supporter hinge axis; and
a second hinge axis holder that covers another surface of the nozzle supporter hinge axis and is coupled to the first hinge axis holder.

14. The shoe care device of claim 13, wherein the first hinge axis holder has a hinge axis holder groove provided on an outer surface, and
wherein the second hinge axis holder has a hinge axis holder hook provided on an outer surface of the second hinge axis holder, the hinge axis holder hook being configured to be fastened to the hinge axis holder groove.

15. The shoe care device of claim 7, further comprising:
a nozzle connector installed separately from the nozzle duct, and having one ed hinged to the inner cabinet and another end hinged to the nozzle.

16. The shoe care device of claim 15, wherein the nozzle connector is disposed under nozzle supporter, and
wherein the nozzle supporter has a nozzle supporter groove provided on a lower surface thereof to correspond to a shape of an upper surface of the nozzle connector.
